# FASCICULE DE BREVET EUROPEEN

(11) **EP 1 620 440 B1**
(45) Date de publication et mention de la délivrance du brevet: **11.03.2009**
(21) Numéro de dépôt: 04742623.4
(22) Date de dépôt: 30.04.2004
(51) Int. Cl.: C07D 513/04, C07D 403/12, C07D 233/88, C07D 263/48, C07D 277/40, C07D 471/04, C07D 498/04

(54) **SYNTHESE DE COMPOSES HETEROCYCLIQUES SUBSTITUES**
SYNTHESE VON SUBSTITUIERTEN HETEROCYCLISCHEN VERBINDUNGEN
SYNTHESIS OF SUBSTITUTED HETEROCYCLIC COMPOUNDS

(30) Priorité: 07.05.2003 FR 0305569
(43) Date de publication de la demande: 01.02.2006
(73) Titulaire: CENTRE NATIONAL DE LA RECHERCHE SCIENTIFIQUE (CNRS), 75794 Paris Cedex 16 (FR); Université de Paris-Sud, 91400 Orsay (FR)
(72) Inventeur: AL MOURABIT, Ali, F-91190 Gif-sur-Yvette (FR); TRAVERT, Nathalie, F-91190 Gif-sur-Yvette (FR); ABOU-JNEID, Robert, F-75012 Paris (FR); GHOULAMI, Said, F-91400 Orsay (FR)
(74) Mandataire: Bredema
(86) Numéro de dépôt international: PCT/FR2004/001059
(87) Numéro de publication internationale: WO 2004/101573

(56) Documents cités:
- US-B1- 6 441 013

## Description

L'invention se rapporte à la synthèse de composés hétérocycliques substitués. Elle vise plus spécialement un procédé de synthèse d'hétérocycles à 5 chaînons de structure H : 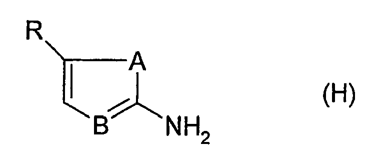 dans laquelle A et B sont des hétéroatomes, A pouvant être substitué selon la nature de l'hétéroatome et R est un groupe de substitution.

On sait que de tels composés sont utilisables pour la synthèse de précurseurs de nombreux produits naturels dotés de propriétés de grand intérêt, notamment, dans le domaine pharmaceutique, en particulier pour la synthèse d'alcaloïdes d'origine marine, de dérivés d'azasucres et d'analogues de ces produits.

De nombreux travaux ont donc porté sur la recherche de voies permettant la synthèse de tels produits. Toutefois, les procédés proposés à ce jour sont complexes, et donc coûteux, et de rendements peu satisfaisants.

Les inventeurs ont constaté que ces problèmes pouvaient être surmontés par ouverture de bi-hétérocycles de structure B-H : 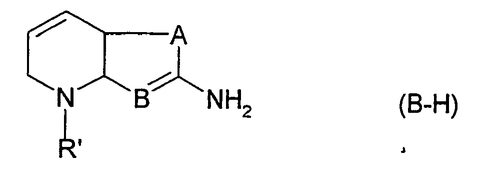 dans laquelle A et B sont tels que définis ci-dessus, et R' est un groupe de substitution.

L'invention a donc pour but de fournir un procédé d'obtention d'hétérocycles à 5 chaînons et deux hétéroatomes, substitués.

Elle vise également l'obtention de ces bicycles à partir de dihydropyridines.

L'invention concerne en outre les nouveaux hétérocycles obtenus et les bicycles intermédiaires.

Elle porte de plus sur les applications de ce procédé et des produits obtenus à la synthèse de produits naturels ou analogues de tels produits.

Le procédé de synthèse de composés hétérocycliques de l'invention est **caractérisé en ce qu**'il comprend l'ouverture d'un composé de formule I : 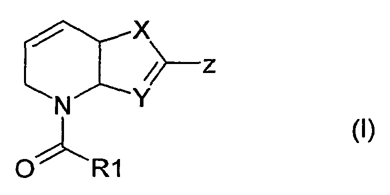 dans laquelle,
- X représente NH, O, S ou un groupe N-p, p étant un groupe protecteur tel que Boc ou Troc,
- Y représente N, O, S
- Z représente NH₂ ou NH-p et
- R₁ représente un radical alkoxy en C₁-C₆, aryloxy, notamment phényloxy, ou un radical pyrrolyle, ces radicaux étant éventuellement substitués, ou de leurs sels, et des isomères de ces composés,
ladite étape d'ouverture étant réalisée dans des conditions conduisant à un hétérocycle de formule II : 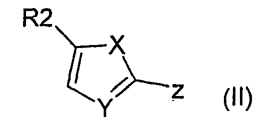 dans laquelle,
- X, Y et Z sont tels que définis ci-dessus, et
- R₂ représente un groupe -CH=CH-CH₂-NH-COR₁ ou 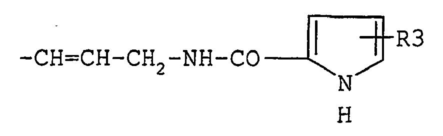
- -R₃ occupant une, deux ou trois positions et représentant un halogène.
   L'étape d'ouverture du bicycle est avantageusement réalisée en milieu alcalin, ou dans un solvant tel que le DMSO, en chauffant au reflux.
   En variante, en particulier lorsque R₁ représente un groupe pyrrolyle, le cas échéant substitué, on fait réagir le bicycle en solution dans un solvant tel que le dichlorométhane avec de l'acide trifluoroacétique, à température ambiante.
   L'étape d'ouverture est réalisée notamment sur un mélange de régioisomères de formule I.
   De manière préférée, on utilise un sel d'un composé de formule I.
   Dans un mode préféré de réalisation de l'invention, les composés de formule I sont des hydro-imidazo-pyridines, X et Y comportant N comme hétéroatomes.
   Il s'agit d'hydro-imidazo-pyridines de formule III : 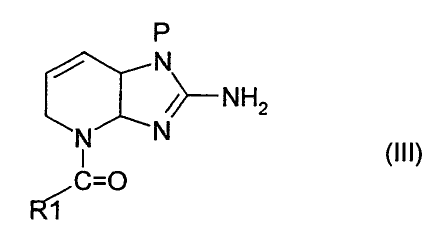
ou de formule IV :

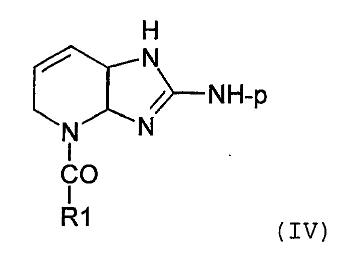

Avantageusement, l'étape d'ouverture selon l'invention est réalisée sur un sel de ces hydro-imidazo-pyridines. Ce sel présente la formule V ou VI :

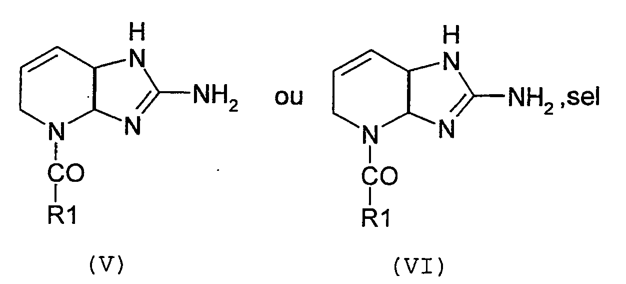

Parmi les sels appropriés, on citera, à titre purement illustratif, les chlorhydrates, acétates, trifluoroacétates.

Dans un autre mode préféré de réalisation de l'invention, les composés de formule I soumis à l'étape d'ouverture sont des hydro-oxazo-pyridines, X représentant l'hétéroatome O.

Des composés préférés répondent à la formule VII :

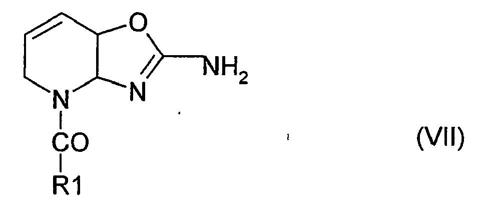

En portant à reflux une solution de ces composés dans un solvant tel que le DMF, on obtient des bicycles de formule VIII.

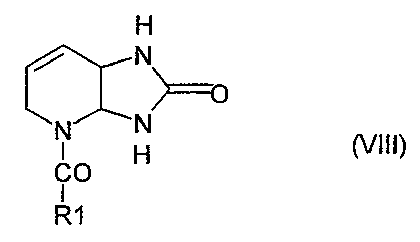

Dans encore un autre mode préféré de réalisation de l'invention, les composés de formule I sont des hydro-thiazo-pyridines, X représentant l'hétéroatome S.
- Les composés de formule II obtenus par ouverture des composés de formule I définis ci-dessus sont substitués par une chaîne allylamine libre ou protégée sous forme de carbamate, -CH=CH-CH₂-NH₂R₁,allylamide -CH=CH-CH₂-NH-COR₁ ou 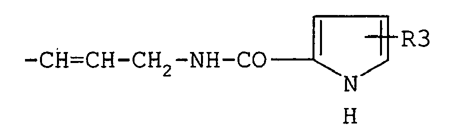
- R₃ occupant une, deux ou trois positions et représentant un halogène.

Selon une autre disposition supplémentaire de l'invention, lorsqu'on souhaite obtenir les composés de formule II sous forme de sels, on les fait réagir avec un acide approprié, tel que mentionné ci-dessus.

Les bicycles de formule I sont avantageusement obtenus par réaction d'une dihydropyridine de formule IX : 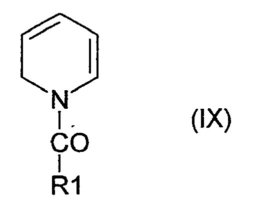 avec un dérivé de formule X : 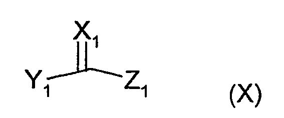 dans laquelle :
- X₁ représente O, S ou NH, et
- Y₁ et Z₁ représentent NH₂.

Cette réaction entre les composés de formules IX et X est avantageusement réalisée en présence de brome dans des solvants organiques tels que DMF et/ou CH₃CN, à température ambiante et sous argon.

Les produits obtenus sont récupérés et purifiés si on le souhaite, par exemple par chromatographie et les isomères sont séparés si souhaité.

L'invention vise également, en tant que nouveaux produits, les composés de formule II et, en tant qu'intermédiaires, les composés de formule I tels que définis ci-dessus.

Les composés de l'invention sont avantageusement utilisables pour la synthèse rapide de précurseurs naturels par exemple d'alcaloïdes d'origine marine, comme l'hyménidine, la chlathrodine et l'oroidine, pour la synthèse de produits analogues d'alcaloïdes d'origine marine comme la girolline, ou pour la synthèse de dérivés d'azasucres, le cas échéant substitués et fonctionnalisés.

L'invention fournit ainsi les moyens pour obtenir par voie de synthèse des précurseurs biogénétiques de produits dotés de propriétés thérapeutiques de grand intérêt, notamment présentant des activités immunosuppressives, anti-tumorales, anti-microbiennes et anti-virales ou encore une activité anti-appétente.

D'autres caractéristiques et avantages de l'invention sont donnés dans les exemples qui suivent.

### Exemple 1 : 1-tert-butylate 4-méthylate de l'acide (cis)-2-Amino-5,7a-dihydro-3aH-imidazo[4,5-b]pyridine-1,4-dicarboxylique (2) et méthylate de l'acide (cis)-2-tert-Butoxycarbonylamino-1,3a,5,7a-hexahydro-imidazo[4,5-b]pyridine-4-carboxylique (3) :

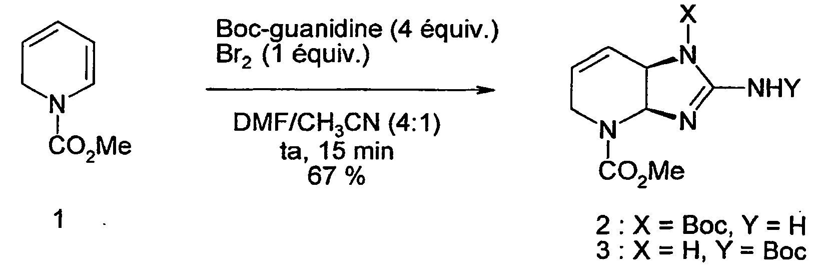

Une solution de Br₂ (0,49 mL, 9,57 mmoles, 1 équiv.) dans du DMF (5 mL) est ajoutée pendant 15 min à une solution de *N*-méthoxycarbonyl-1,2-dihydropyridine 1 (1,33 g, 9,57 mmoles, 1 équiv.) et de Boc-guanidine (6,1 g, 38,3 mmoles, 4 équiv.) dans un mélange de DMF (15 mL) et de CH₃CN (5 mL), à température ambiante, et sous argon. La réaction étant terminée dès la fin de l'addition du brome, le solvant est évaporé à 50°C et le brut (11 g) chromatographié sur gel de silice à l'aide du dichlorométhane saturé avec de l'ammoniac. Les régioisomères 2 et 3 sont obtenus en mélange, sous la forme d'un solide jaune hygroscopique (1,9 g, 67 %). Les deux produits peuvent être facilement séparés par chromatographie sur couche de silice épaisse et analysés séparément :

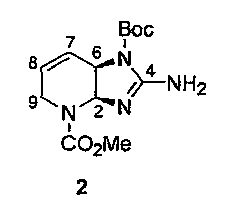

Régioisomère **2 :** solide jaune ; F = 75,5°C (déc.) ; **¹H RMN** (300 MHz, CD₃OD) : δ = 1,55 (s, 9H, (CH₃)₃), 3,63 (m, 4H, 9-Hₐ), 3,75 (s, 6H, OCH₃), 4,19 (dd, *J* = 18 et 2 Hz, 1H, 9-H_{b}), 4,63 (m, 1H, 6-H), 5,82 (m, *J* = 2 et 11 Hz, 1H, 7-H), 6,07 (m large, 2H, 2-H et 8-H) ; **¹³C NMR** (75,5 MHz, CD₃OD) : δ = 28,3 (CH₃)₃, 39,6 (C-9), 53,5 (C-3'), 55,0 (C-6), 70,0 (C-2), 84,4 (C-8"), 122,0 (C-7), 129,0 (C-8), 153,0, 156,0, 158,0 (C-4, co-carbamate) ; **MS(ES) :** *m*/*z* 296,9 [*M*+H]⁺, 196,8 [*M*+H-Boc]⁺; **MSHR,** calculé C₁₃H₂₀O₄N₄ [*M*+H]⁺ : 297,15628, trouvé : 297,15605.

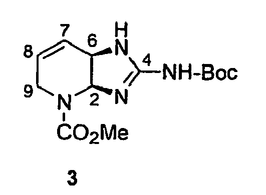

Régioisomère **3** : solide jaune ; F = 117°C (déc.) ; **RMN ¹H** (300 MHz, CD₃OD) : δ = 1,47 (s, 9H, (CH₃)₃), 3,47 (d, 18Hz, 1H, 9-Hₐ), 3,75 (s, 3H, OCH₃), 4,12 (d large, *J* = 8 Hz, 1H, 6-H), 4, 31 (d large, *J* = 18Hz, 2H, 9-H_{b}), 5,62 (dd, *J* = 2 et 11 Hz, 1H, 7-H), 5,85 (s large, 1H, 8-H), 6,46 (s large, 1H, 2-H) ; **NMR ¹³C** (75, 5 MHz, CD₃OD) : δ = 28,4 (CCH₃), 39, 1 (C-9), 53,6 (OCH₃), 55,9 (C-6), 70,2 (C-2), 84,8 C(tBu), 124,3 (C-8), 127,5 (C-7) ; **MS(ES) :** *m*/*z* 296,9 [*M*+H]⁺, 196,8 [*M*+H-Boc]⁺; **MSHR,** calculé C₁₃H₂₀O₄N₄ [*M*+H]⁺ : 297,15628, trouvé : 297,15769.

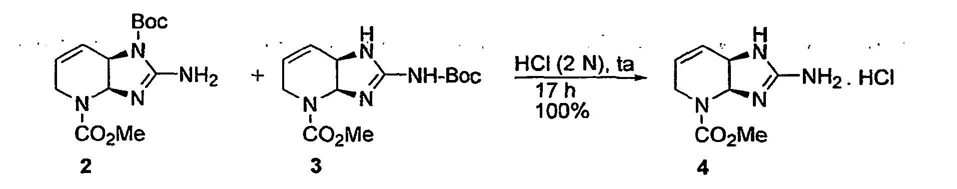

### Exemple 2 : chlorhydrate du méthylate de l'acide (cis)-2-amino-1,3a,5,7a-tétrahydro-imidazo[4,5-b]pyridine-4-carboxylique 4

Une solution du mélange de produits **2** et **3** (124 mg, 0,42 mmole) dans HCl 2M (9 mL) est laissée sous agitation pendant 17 h à température ambiante, puis concentrée à sec. Le produit **4** est obtenu sous la forme de chlorhydrate semi-solide marron (99 mg, 0,42 mmole, quantitatif).

### Exemple 3 : synthèse du produit déprotégé 4 à partir de la dihydropyridine et sans purification des régioisomères

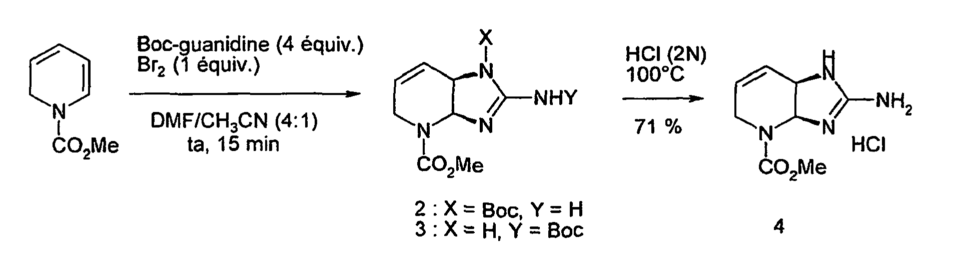

Une solution de Br₂ (0,73 mL, 14,3 mmoles, 1 équiv.) dans du DMF (5 mL) est ajoutée pendant 15 min à une solution de *N*-méthoxycarbonyl-1,2-dihydropyridine 1 (2 g, 14,3 mmoles, 1 équiv.) et de Boc-guanidine (9,15 g, 57,5 mmoles, 4 équiv.) dans un mélange de DMF (10 mL) et de CH₃CN (5 mL) à froid (0-5°C) et sous argon. La réaction étant terminée dès la fin de l'addition du brome, le mélange réactionnel est versé sur de l'eau glacée (600 mL). La phase aqueuse est ensuite extraite par du DCM (3x200 mL). Le brut obtenu (3,5 g) est solubilisé dans un minimum de méthanol, puis addition d'une solution d'acide chlorhydrique 2N (10 mL). La solution est portée à reflux dans .... pendant 5 min. Après refroidissement, le mélange réactionnel est lavé par l'éther, puis évaporé à sec pour donner 2,4 g du composé déprotégé **4,** avec un rendement de 71 % sur les deux étapes. 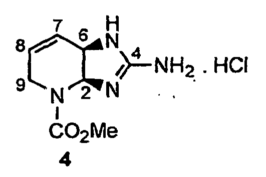 **RMN ¹H** (300 MHz, CD₃OD) : δ = 3,69 (d large, *J* = 19 Hz, 1H, 9-H_{b}), 3,79 (s, 3H, OCH₃), 4,25 (dd large, *J* = 19 et 4 Hz, 1H, 9-Hₐ), 4,47 (d large, *J* = 9 Hz, 1H, 6-H), 5,74 (qd, J = 10 Hz, 1H, 7-H), 6,08 (dd large, *J* = 10 et 4 Hz, 1H, 8-H), 6,39 (d, *J* = 8 Hz, 1H, 2-H) ; **NMR ¹³C** (75,5 MHz, CD₃OD) : δ = 39,7 (C-9), 52,0 (C-6), 54,0, OCH₃, 66,0 (C-2), 123,0 (C-7), 128,0 (C-8), 157,0, 160,0 (co-carbamate, C-4 ; **MS(ES) :** *m*/*z* 196,8 [*M*+H]⁺; **MSHR,** calculé C₈H₁₂O₂N₄ [*M*+H]⁺ : 197,10385, trouvé : 197,10314.

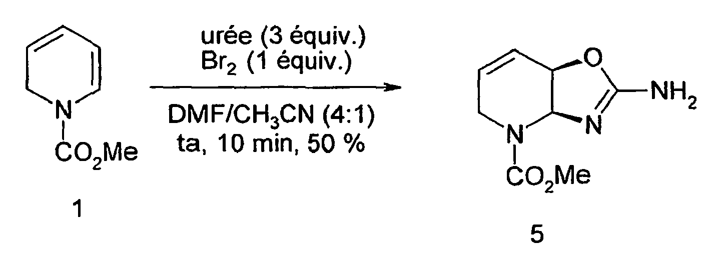

### Exemple 4 _{:} (cis)-2-amino-5,7a-dihydro-3aH-oxazolo[4,5-b]pyridine-4-carboxilic acid methyl ester 5

Une solution de Br₂ (1 mL, 19,3 mmoles, 1 équiv.) dans du DMF (5 mL) est ajoutée pendant 20 min à une solution de *N*-méthoxycarbonyl-1,2-dihydropyridine **1** (2,68 g, 19,3 mmoles, 1 équiv.) et d'urée (2,3 g, 38,6 mmoles, 3 équiv.) dans un mélange de DMF (19 mL) et de CH₃CN (6 mL), à température ambiante, et sous argon. La réaction étant terminée dès la fin de l'addition du brome, le mélange réactionnel est concentré à sec. Le résidu est solubilisé dans du méthanol (30 mL), versé sur de l'eau glacé (150 mL), puis filtré sur Büchner. Le filtrat est alcanilisé (Na₂CO₃ 5 %) jusqu'à l'obtention d'un pH supérieur à 10. Après extraction par le dichlorométhane (4x100 mL), la phase aqueuse est saturée ave du NaCl puis re-extraite par le dichlorométhane (4x100 mL). Après séchage sur MgSO₄ et évaporation du solvant, le solide obtenu (5,94 g) est chromatographié sur gel de silice à l'aide du dichlorométhane (DCM) saturé à l'ammoniac puis DCM saturé par NH₃ : MeOH, 98:2. Le produit est obtenu sous la forme d'un solide beige (1,9 g, 50 %). 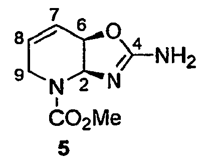 Solide beige, F = 176° (déc.) ; **RMN ¹H** (300 MHz, CD₃OD) : δ = 3,65 (d large, *J* = 18 Hz, 1H, 9-Hₐ), 3,75 (s, 3H, 3'-H), 4,18 (dd, *J* = 18 et 4 Hz, 1H, 9-H_{b}), 5,0 (dd, *J* = 8 et 4 Hz, 1H, 6-H), 5,7 (m, *J* = 4 et 11 Hz, 1H, 7-H), 6,1 (dd, *J* = 11 et 4 Hz, 1H, 8-H), 6,27 (d large, *J* = 8 Hz, 1H, 2-H) ; **RMN ¹H** (300 MHz, *d*⁶-DMSO) : δ = 3,50 (d large, *J* = 18 Hz, 1H, 9-Hₐ), 3,63 (s, 3H, OCH₃), 4,04 (dd large, *J* = 18 et 4 Hz, 1H, 9-H_{b}), 4,91 (dd, *J* = 8 et 4 Hz, 1H, 6-H), 5,65 (d large, *J* = 11 Hz, 1H, 7-H), 6,1 (m, 4H, 2-H, 8-H, NH₂) ; **RMN ¹³C** (75,5 MHz, *d*⁶-DMSO) : δ = 38,3 (C-9), 52,5 (OCH₃), 70,7 (C-6), 72,6 (C-2), 122,3 (C-7), 129,0 (C-8), 155,4 (co-carbamate, 160,6 (C-4) ; **MS(ES) :** *m*/*z* 197,9, [*M*+H]⁺; **MSHR,** calculé pour C₈H₁₁O₃N₃ [*M*+H]⁺ : 198, 08787, trouvé : 198, 08771 ; **Anal. Elem. pour C₈H₁₁O₃N₃ :** *calculé* C(48,73 %), H(5,58 %), N(21,32 %), O(24,37 %) ; *trouvé* C(48,49 %), H(5,67 %), N(21,32 %), O(24,27 %).

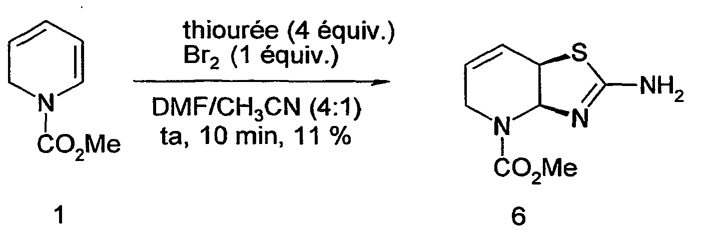

### Exemple 5 : (cis)-amino-5,7a-dihydro-3aH-thiazolo[4,5-b]pyridine-4-carboxilic acid methyl ester 6

Une solution de Br₂ (0, 71 mL, 13,9 mmoles, 1 équiv.) dans du DMF (5 mL) est ajoutée pendant 10 min à une solution de *N*-méthoxycarbonyl-1,2-dihydropyridine 1 (1,92 g, 13 mmoles, 1 équiv.) et de thiourée (3,95 g, 52 mmoles, 4 équiv.) dans le DMF (15 mL)/CH₃CN (5 mL), à température ambiante et sous argon. La réaction étant terminée dès la fin de l'addition du brome, le mélange réactionnel est concentré deux fois environ, versé sur de l'eau glacée (200 mL), puis filtré sur Büchner. Le filtrat est alcanilisé (Na₂CO₃ 5 %) jusqu'à l'obtention d'un pH supérieur à 10, puis saturé avec du NaCl. Après extraction au DCM, séchage sur MgSO₄ et évaporation du solvant, le liquide obtenu (10,8 g) est chromatographié sur gel de silice DCM (sat. NH₃), puis DCM (sat NH₃/MeOH, 2 %). Le produit **6** (R_{f} = 0,39, DCM/MeOH(NH₃), 98:2) est obtenu sous la forme d'un solide blanc (0,32 g, 11 %).

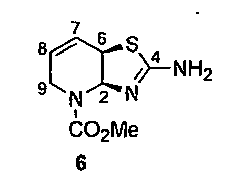

**RMN ¹H** (300 MHz, acétone-d₆) : δ = 3, 64 (m large, 9-Hₐ), 3,70 (s, 3H, OCH₃), 4,17 (d, *J* = 17 Hz, 1H, 9-H_{b}), 4,30 (m, 6-H), 5,50 (m, *J* = 2 Hz, *J* = 10 Hz, 1H, 7-H), 5,76 (m, 1H, 8-H), 6, 19 (m, 1H, 2-H). **RMN ¹³C** (75, 5 MHz, DMSO-d₆) : δ = 38,6 (C-9), 47,5 (C-6), 52,6 (OCH₃), 79,0 (C-2), 123,5 (C-7), 125,7 (C-8), 155,2 (C-4), 156,9 (CO carbamate). **MS(IE) :** *m*/*z* = 213[*M*]⁺.

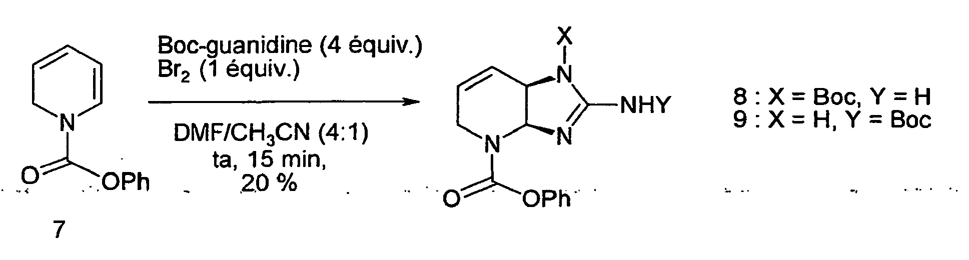

### Exemple 6 : (cis)-2-amino-5,7a-dihydro-3aH-imidazo[4,5-b]pyridine-1,4-dicarboxilic acid 1-tert-butyl ester 4-phenyl ester 8 et (cis)-2-tert-butoxycarbonylamino-1,3a,5,7a-hexahydro-imidazo[4,5-b]pyridine-4-carboxylic acid phenyl ester 9

Une solution de Br₂ (0,36 mL, 7 mmoles, 1 équiv.) dans du DMF (5 mL) est ajoutée sur une période de 15 min à une solution de *N*-phényloxycarbonyl-1,2-dihydropyridine 7 (1,42 g, 7 mmoles, 1 équiv.) et de Boc-guanidine (4,5 g, 28 mmoles, 4 équiv.) dans DMF (15 mL)/CH₃CN (5 mL), à température ambiante et sous argon. La réaction étant terminée dès la fin de l'addition du brome, lee solvant est évaporé sous vide et le brut (2 g) chromatographié sur gel de silice (DCM sat. NH₃). Les deux régiosomères (R_{f} = 0,40, DCM(NH₃)) sont obtenus sous la forme d'un solide blanc (1,9 g, 6,42 mmoles, 20 %). Les deux produits **8** et **9** sont facilement purifiés par chromatographie sur couche épaisse.

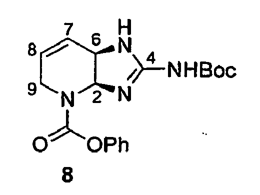

### (cis)-2-amino-5,7a-dihydro-3aH-imidazo[4,5-b] pyridine-1,4-dicarboxilic acid 1-tert-butyl ester 4-phenyl ester 8 :

**RMN ¹H** (300 MHz, CD₃OD): δ = 1,56 (s, 9H, (CH₃)₃), 3,78 (dd, 1H, 9-Ha, *J* = 18 Hz), 4,33 (dd, *J* = 18 Hz, 1H, 9-H_{b}), 4,73 (m, 1H, 6-H), 5,88 (d large, 1H, *J* = 10 Hz, 7-H), 6,11 (m, 1H, 8-H), 6,24 (dd, *J* = 8 Hz, 1H, 2-H), 7,26 (m, 5H, H-phényl) ; **RMN ¹³C** (75,5 MHz, CD₃OD) δ = 28,7 C(CH₃)₃, 40,1 (C-9), 55,6 (C-6), 70,5 (C-2), 84,8 (CtBu), 122,8 (C-8), 122,8 (C-8), 127,1 (C-7), 123,3, 130,7 (C-Ph), 153,0 (C-4) ; **MS(IE) :** *m*/*z* 358 [*M*]⁺, 257 [*M*-Boc]⁺.

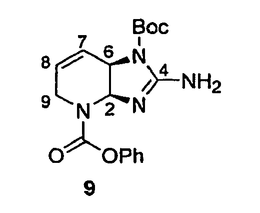

### Phénylate de l'acide (cis)-2-tert-butoxycarbonylamino-1,3a,5,7a-tétrahydro-imidazo[4,5-b]pyridine-4-carboxylique 9 :

**RMN ¹H** (300 MHz, CD₃OD) : δ = 1,46 (s, 9H, (CH₃)₃), 3,57 (dd, *J* = 19 Hz, 1H, 9-Hₐ), 4,18 (d, *J* = 8 Hz, 1H, 6-H), 4,40 (dd, *J* = 19Hz, H, 9-H_{b}), 5,68 (m, 1H, 7-H), 5,87 (m, 1H, 8-H), 6,55 (dd, *J* = 8 Hz, 1H, 2-H), 7,22 (m, 5H, H-phényl) ; **RMN ¹³C** (75,5 MHz, CD₃OD) : δ = 28,9 (CH₃)₃, 40,1 (C-9), 56,5 (C-6), 70,5 (C-2), 85,8 (CtBu), 122,8 (C-Ph), 124,8 (C-8), 127,1 (C-7), 128,3, 130,7 (C-Ph) ; **MS(IE) :** *m*/*z* 358 [*M*]⁺, 257 [*M*-Boc]⁺.

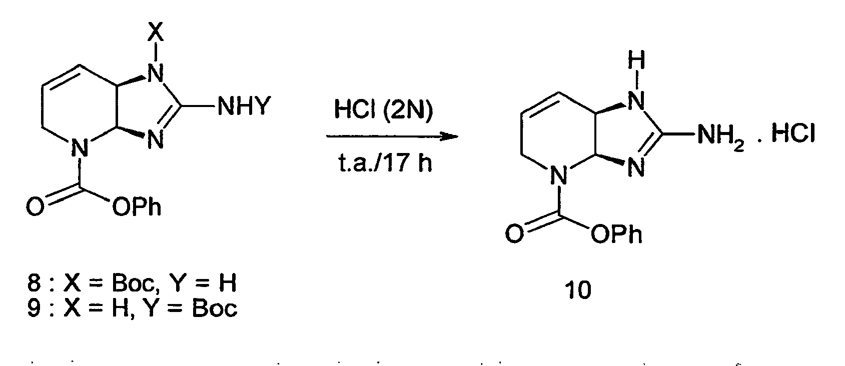

### Exemple 7 : chlorhydrate du phénylate de l'acide (cis)-2-amino-1,3a,5,7a-tétrahydro-imidazo[4,5-b]pyridine-4-carboxylique 10

Une solution d'un mélange de produits **8** et **9** (54 mg, 0,15 mmole) dans HCl (2 M, 5 mL) est agitée pendant 17 h à température ambiante, puis concentrée à sec. Le produit **10** (Rf = 0.5, acétone/AcOEt/eau/HCOOH, 30 : 50 : 5 : 5) est obtenu sous forme d'un solide jaune-marron (37 mg, 84 %).

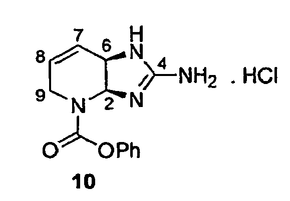

**RMN ¹H** (300 MHz, CD₃OD) : δ = 3,84 (dd, *J* = 18 Hz, 1H, 9-Hₐ), 4,36 (dd, *J* = 18 Hz, H, 9-H_{b}), 4,53 (s large, 1H, 6-H), 5,78 (dd, 1H, et 5 Hz, 7-*H* = 10), 6,13 (m large, 1H, 8-H), 6,50 (m large, 1H, 2-H) 7,32 (m, 5H, H-phényl) ; **RMN ¹³C** (75,5 MHz, CD₃OD) : δ = 40,2 (C-9), 52,1 (C-6), 66,6 (C-2), 123,2 (C-8), 127,4 (C-7), 130,8, 130,9 (C-Ph), 152,7 (C-4), 160,5 (carbamate) ; **MS(ES) :** *m*/*z* 259,2 [*M*+H]⁺.

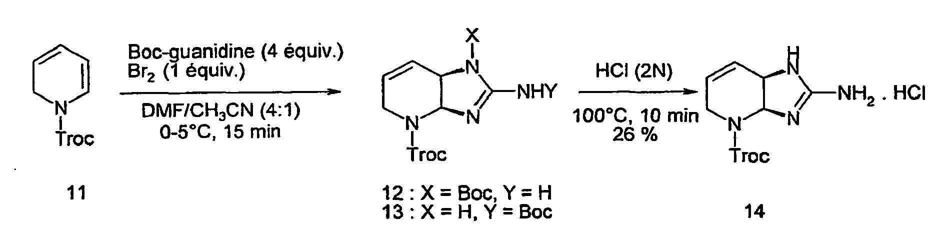

### Exemple 8 : chlorhydrate du 2,2,2-trichloroéthylate de l'acide (cis)-2-amino-1,3a,5,7a-tétrahydro-imidazo[4,5-b]pyridine-4-carboxylique

Une solution de Br₂ (0,79 mL, 15,5 mmoles, 1 équiv.) dans DMF (5 mL) est ajoutée sur une période de 15 min à une solution de *N*-trichloroéthylcarbonyl-1,2-dihydropyridine 11 (4 g, 15,5 mmoles, 1 équiv.) et de Boc-guanidine (10 g, 62,0 mmoles, 4 équiv.) dans DMF (10 mL)/CH₃CN (5 mL) à froid (0-5°C) et sous argon. La réaction étant terminée dès la fin de l'addition du brome, le mélange réactionnel est versé sur de l'eau glacée (600 mL). La phase aqueuse est ensuite extraite par du DCM (3x200 mL). Les phases organiques sont réunies, puis lavées à l'eau et évaporées à sec. Le brut obtenu (3,5 g) est solubilisé dans un minimum de méthanol, puis porté à reflux dans 10 mL d'une solution d'acide chlorhydrique 2N pendant 10 minutes. Après refroidissement, le mélange réactionnel est lavé par l'éther puis évaporé à sec pour donner 1,4 g du produit déprotégé **14** avec un rendement de 26 % sur les deux étapes.

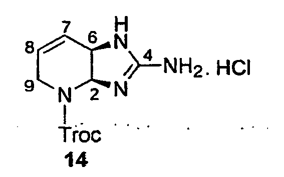

**RMN ¹H** (250 MHz, DMSO-d₆) : δ = 3, 74 (d large, *J* = 17 Hz, 1H, 9-Hₐ), 4,18 (d large, *J* = 17 Hz, H, 9-H_{b}), 4,50 (d large, *J* = 8 Hz, 1H, 6-H), 4,95 (m, 2H, H-Troc), 5,74 (d, *J* = 9 Hz, 1H, 7-H), 6,07 (m, 1H, 8-H), 6,24 (d, *J*₂₋₆ = 8 Hz, 1H, 2-H) ; **NMR ¹³C** (62,5 MHz, CD₃OD) : δ = 40,1 (C-9), 51,8 (C-6), 66,3 (C-2), 76,6 (CH₂-Troc), 122,9 (C-8), 127,0 (C-7), 156,0 (C-4), 160,0 (carbamate) ; **MS(ES) :** *m*/*z* 313,0 ; 315,0 ; 317,0 [*M*]⁺.

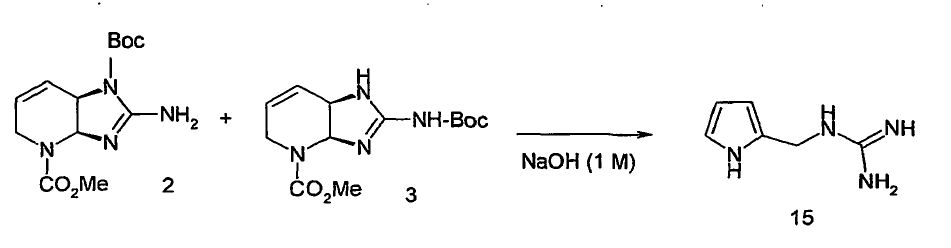

### Exemple 9 :N-(1H-pyrrol-2-yl)-guanidine formate 15

Une solution du mélange d'isomères **2** et **3** (131 mg, 0,44 mmole) dans NaOH 1M (10 mL) est chauffée au reflux pendant 20 min. Après refroidissement à température ambiante, neutralisation à l'aide d'une solution HCl 1M, le mélange réactionnel est concentré à sec. Le résidu solide est trituré par du méthanol, puis filtré. Le filtrat est concentré à sec (333 mg), puis chromatographié sur gel de silice (acétone/AcOEt/H₂O/HCO₂H, 360:600:12:12, puis 300:500:50:50) pour donner 54 mg de **15** (R_{f} = 0.44, acétone/AcOEt/H₂O/HCO₂H, 30:50:5:5) avec 67 % de rendement.

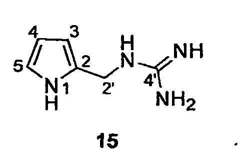

**RMN ¹H** (300 MHz, CD₃OD) : δ = 3,34 (s, 2H, 2'-H), 6,04 (m, 1H, 3-H), 6,09 (m, 1H, 4-H), 6,73 (app s, 1H, 5-H), 8,58 (s large, 1H, HCO₂D) ; **RMN ¹³C** (75,5 MHz, CD₃OD) : δ = 39,6 (C-2'), 108,4, 108,9 (C-3, C-4), 119,5 (C-5), 126,6 (C-2), 158,4 (C-4') ; **MS(ES) :** *m*/*z* 139,2 [*M*+H]⁺ ; **HRMS,** calculé C₆H₁₀N₄ [*M*+H]⁺ : 139,09837, trouvé : 139,09843.

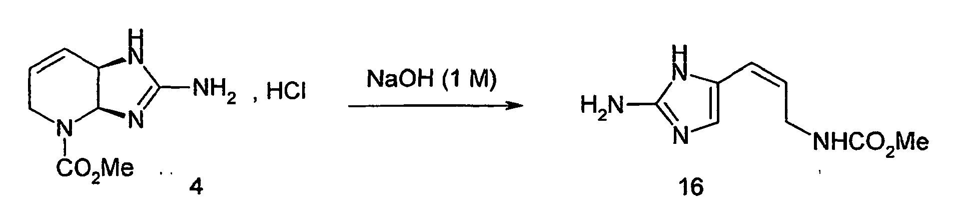

### Exemple 10 : méthylate de l'acide [3-(2-amino-2,3-dihydro-1H-imidazo-4-yl)-allyl]-carbamique 16

A 4 ml d'une solution NaOH (1 mL), on ajoute 0,111 g (0,47 mmole) de l'ester méthylique de l'acide 2-amino-1,3a,5,7a-tetrahydro-imidazo[4,5-b]pyridine-4 carboxylique **4.** Le mélange réactionnel est porté à reflux sous agitation pendant 5 minutes (réaction contrôlée par CCM). Après refroidissement, on ajoute une solution tampon de phosphate (pH = 7) (20 mL), et le mélange est extrait au BuOH jusqu'à épuisement. Les phases organiques sont regroupées et évaporées à sec pour donner 0,079 g d'amine allylique **16** sous forme d'un solide marron avec un rendement de 85 %.

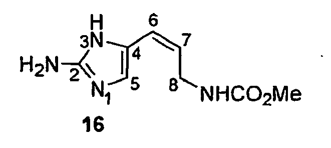

**RMN ¹H** (300 MHz, d₁-TFA) : δ = 3,88 (s, 3H, OMe), 4,09 (dd, *J* = 7 et 2 Hz, 2H, 8-H), 5,87 (dt, *J* = 7 Hz, 12 Hz, 1H, 7-H), 6,23 (d, *J* = 12 Hz, 1H, 6-H), 6,73 (s, 1H, 5-H) ; **RMN ¹H** (300 MHz, CD₃OD) : δ = 3,64 (s, 3H, OMe), 3,96 (dd, *J* = 7 et 1 Hz, 2H, 8-H), 5,37 (dt, *J* = 7 et 11 Hz, 1H, 7-H), 6,15 (d, *J* = 11 Hz, 1H, 6-H), 6,51 (s, 1H, 5-H) ; **RMN ¹³C** (75,5 MHz, CD₃OD) : δ = 41,0 (C-8), 52,5 (OMe), 118,0 (C-5), 121,0 (C-6), 125,0 (C-7), 130,0 (C-4), 151,0, 160,0 (C-2 et CO carbamate) ; **MS(ES) :** *m*/*z* 197,1 [*M*+H]⁺ ; **MSHR,** calculé C₈H₁₂O₂N₄ [M+H]⁺ : 197,10385, trouvé : 197,10395.

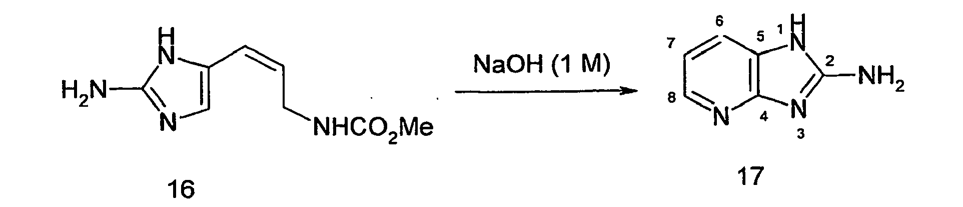

### Exemple 11 :1H-imidazo[4,5-b]pyridin-2-ylamine 17

20 mg (0,1 mmole) de l'amine allylique 16 cis sont solubilisés dans une solution NaOH 1M (2 mL). Le mélange est laissé à température ambiante pendant 48 h. Cette solution est extraite au BuOH (3x20 mL). Les phases butanoliques sont réunies et évaporées à sec. Le résidu est repris par un minimum de méthanol, puis purifié sur des plaques préparatives de gel de silice en utilisant le mélange DCM/MeOH (NH₃), 8/2 comme éluant. Le composé 17(4 mg) est obtenu avec un rendement de 31 %.

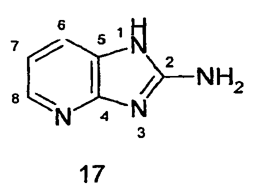

***RMN* ¹H** (300 MHz, CD₃OD) : δ = 6, 69 (dd, *J* = 7 et = 7Hz, 1H, H-7), 7,41 (d, *J* = 7 Hz, 1H, H-6), 7,88 (d, *J* = 6,5 Hz, 1H, H-8). **RMN ¹³C** (75,5 MHz, CD₃OD) : δ = 117,3 (C-7), 119,4 (C-6), 141,0 (C-8), **MS(ES) :** *m*/*z* 135,2 [*M*+H]⁺.

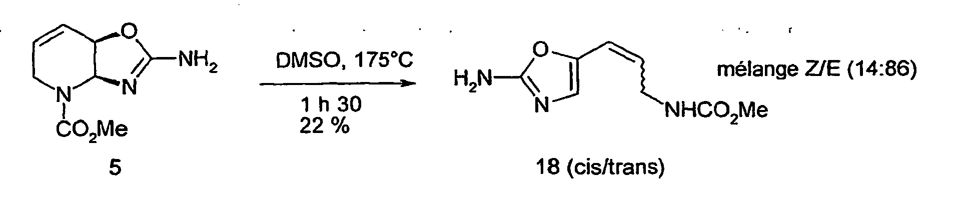

### Exemple 12 : [méthylate de l'acide 3-(2-amino-oxazol-5-yl)-allyl]-carbamique 18

Une solution du produit **5** (1,2 g, 6,1 mmoles, 0,08 M) dans du DMSO (75,5 mL) est chauffée à 175°C pendant 1 h 30. Après refroidissement à température ambiante, le mélange réactionnel est versé sur de l'eau (200 mL), alcalinisé (Na₂CO₃ 5 %) jusqu'à un pH supérieur à 10, puis extrait au DCM (4x150 mL). La phase aqueuse est alors saturée avec NaCl, puis extraite au DCM (4x150 mL). Les phases organiques sont réunies, séchées (MgSO₄), puis évaporées. Le brut obtenu (6,6 g) est alors purifié par chromatographie sur gel de silice (DCM (sat. NH₃)), puis DCM (sat. NH₃)/MeOH, 98/2). Le produit **18** (R_{f} = 0, 36, DCM/MeOH(NH₃): 98/2) est obtenu sous la forme d'un semi-solide marron (265 mg, 22 %), mélange inséparable d'isomères Z/E (14:86).

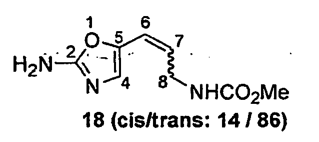

**(E)-18 : RMN ¹H** (300 MHz, CD₃OD) : δ = 3,64 (s, 3H, OMe), 3,80 (d, *J* = 7 Hz, 2-H, 8-H), 5,85 (dt, *J* = 6 et 16 Hz, 1H, 7-H), 6,22 (dd, *J* = 16 et 1 Hz, 1H, 6-H), 6,54 (s, 1H, 4-H) ;. **RMN ¹³C** (75,5 MHz, CD₃OD) : δ = 43,3 (C-8), 52,6 (OMe), 117,4 (C-6), 123,8 (C-7), 124,4 (C-4), 144,7 (CO carbamate), 159,5, 163,1 (C-2, C-5). (Z)18 : **RMN ¹H** (300 MHz, CD₃OD) : δ = 3,64 (s, 3H, 12-H), 4,06 (d large, *J* = 6 Hz, 1-H, 8-H), 5,36 (dt, *J* = 6 et 12 Hz, 1H, 7-H), 6,09 (dd, *J* = 12 et 1 Hz, 1-H, 6-H), 6,63 (s, 1-H, 4-H). **RMN ¹³C** (75,5 MHz, CD₃OD) : δ = 40,9 (C-8), 52,5 (OMe), 115,8 (C-6), 125,6 (C-7), 126,7 (C-4), 144,5 (CO carbamate) (C-2, C-5, non détectés). **MS(ES) :** *m*/*z* 197,8 [*M*+H]⁺.

### Exemple 12bis : méthylate de l'acide [3-(2-imino-2,3-dihydro-thiazol-5-yl)-allyl]-carbamique

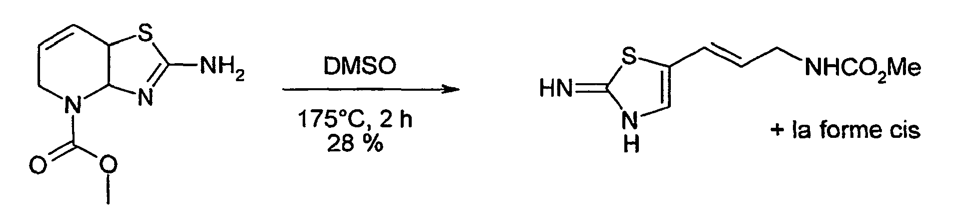

Le composé **6** (0,070 g, 0,32 mmole) est chauffé dans du DMSO (5 mL) à 175°C pendant 2 h. Après refroidissement à température ambiante, le mélange réactionnel est versé sur de l'eau glacée (100 mL), alcalinisé (Na₂CO₃ 5 %) jusqu'à un pH supérieur à 10, puis extrait au DCM (3x150 mL). Les phases organiques sont réunies, lavées avec une solution saturée de NaCl, séchées (MgSO₄), puis évaporées à sec. Le brut obtenu (0,06 g) est alors purifié par chromatographie sur couches épaisses de gel de silice DCM (sat. NH₃/MeOH, 95:5). Le produit (Rf = 0,49, DCM/MeOH/NH₃ vap., 95:5) est obtenu sous la forme d'un semi-solide marron (20 mg, 0,09 mmole, 28 %), mélange inséparable d'isomères E/Z (q:1) d'après la RMN¹H.

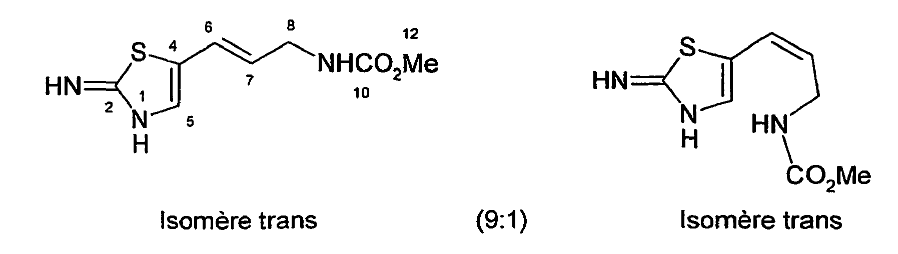

### [3-(2-imino-2,3-dihydro-thiazol-5-yl)-allyl]-carbamic acid methyl ester :

(Isomère E) : **RMN ¹H** (300 MHz, CDCl₃) : δ = 3, 64 (s, 3H, H-12), 3,8 (d, *J* = 5 Hz, 2H, H-8), 5,57 (dt, *J* = 6 et 15 Hz, 1H, H-7), 6,45 (d, *J* = 15 Hz, 1H, H-6), 6,85 (s, 1H, H-4) ; **RMN ¹³C** (75,5 MHz, CDCl₃) : δ = 42,4 (C-8), 51,9 (C-12), 122,0 (C-6), 124,1 (C-7), 125,8 (C-4), 136,4 (C-5), 157,4 (C-10), 168,1 (C-2). **MS(ES) :** *m*/*z* = 214[*M*+H]⁺ ; MS (HR) (ES) : calculé C₈H₁₁N₃O₂S, trouvé 214.0650. [M+H]⁺, IR (CHCl₃, cm⁻¹): 1699, 1636, 1536, 1503, 1023, 949.

**(Isomère Z) : RMN ¹H** (300 MHz, CDCl₃) : δ = 3,64 (s, 3H, H-12), 3,77 (d, *J* = 5 Hz, 2H, H-8), 5,36 (dt, *J* = 6 et 11 Hz, 1H, H-7), 6,37 (d, *J* = 11 Hz, 1H, H-6), 6,93 (s, 1H, H-4).

(Isomère Z non isolé, valeurs prises sur le même spectre de l'isomère E).

### Exemple 13 : méthylate de l'acide (cis)-2-oxo-1,2,3,3a,5,7a-hexahydro-imidazo[4,5-b]pyridine-4-carboxylique 19

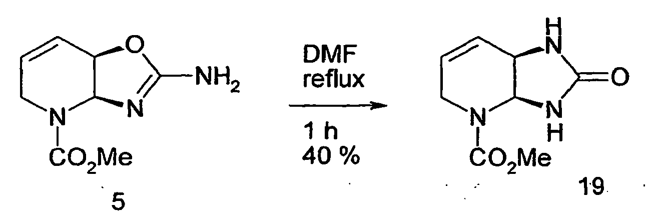

Une solution de **5** (200 mg, 1 mmole) dans du DMF (10 mL) est portée à reflux pendant 1 h. Après évaporation du solvant, le brut (280 mg) est purifié par chromatographie sur gel de silice (Et₂O/MeOH, 93:7). Le produit **19** (R_{f} = 0.36, Et₂O/MeOH, 92:8) est obtenu sous la forme d'un solide jaune (81 mg, 40%).

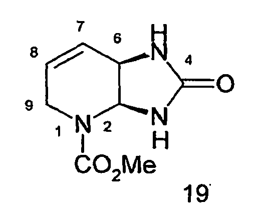

**RMN ¹H** (250 MHz, *d*⁶-DMSO) : δ = 3, 55 (d large, 1H, 9-Hₐ), 3,65 (s, 3H, OCH₃), 4, 06 (m, 2H, 9-H_{b}, 6-H), 5,56 (m, 1H, 7-H), 5,86 (m, 2H, 8-H, 2-H), 6,52 (s large, NH), 6,76 (s large, NH). **RMN ¹H** (250 NHz, CD₃OD) : δ = 3,67 (d large, 1H, 9-Hₐ), 3,76 (s, 3H, OCH₃), 4,17 (m, 2H, 9-H_{b}, 6-H), 5,66 (m, 1H, 7-H), 5,95 (m, 1H, 8-H), 6,14 (d large, *J* = 8 Hz, 1H, 2-H). **RMN ¹³C** (75,5 MHz, *d*⁶-DMSO) : δ = 38,2 (C9), 48,0 (C6), 52,7 (OCH₃), 61,7 (C2), 123,9 (C-8), 124,3 (C7), 155,3 (CO carbamate), 160,98 (C4). **MS(ES) :** *m*/*z* 219,9 [*M*+Na]⁺.

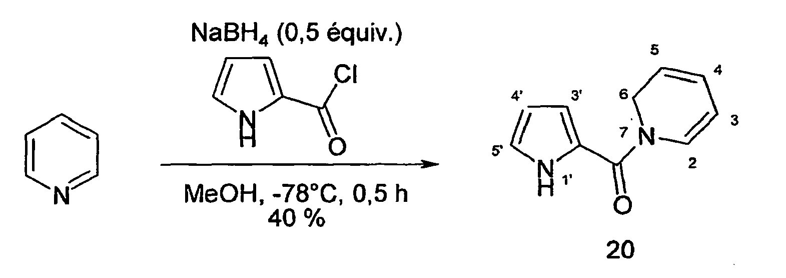

### Exemple 14 : (2H-pyridin-1-yl)-(1H-pyrrol-2-yl)-méthanone 20

L'acide pyrrolique (1,11 g, 10,0 mmoles, 1 équiv.) est placé sous argon, en suspension dans du toluène (20 mL). Le chlorure d'oxalyle (2 mL, 23,0 mmoles, 2,3 équiv.) est ajouté par un goutte à goutte rapide, puis une goutte de DMF, ce qui provoque le dégagement gazeux et la dissolution du produit. Après une nuit à température ambiante, le milieu réactionnel est concentré sous pression réduite et séché à la pompe pendant 2 h. Le chlorure d'acide ainsi obtenu est repris dans du dichlorométhane (10 mL) et introduit dans un bicol, sous argon. La pyridine (0,8 mL, 10,0 mmoles, 1 équiv.) est ajoutée à cette solution. Après 1 h à température ambiante, le précipité formé est séché. Il est repris dans du méthanol (10 mL), refroidi à -78°C et additionné de NaBH₄ (0,19 g, 5,0 mmoles, 0,5 équiv.). Le milieu réactionnel est agité à la même température pendant 30 min, puis il est versé sur de l'eau glacée (50 mL) et extrait par l'éther diéthylique (2x50 mL). La phase organique est séchée sur sulfate de magnésium, puis concentré sous pression réduite. Le résidu est purifié par chromatographie sur colonne de gel d'alumine, élué par le dichlorométhane. La dihydropyridine **20** est obtenue avec un rendement de 39 % (0,68 g).

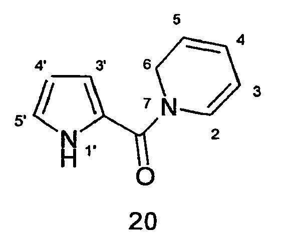

**RMN ¹H** (300 MHz, CDCl₃) : δ = 9,70 (sl, 1H) ; 7,08 (d, *J* = 7,6 Hz, 1H, 2-H) ; 6,99 (m, 1H, 5'-H) ; 6,67 (m, 1H, H3') ; 6,30 (m, 1H, H4') ; 5,98 (m, 1H, H4) ; 5,70 (m, 1H, H5) ; 5,38 (dd, *J* = 7,6 et 7,6 Hz, 1H, 3-H) ; **RMN ¹³C** (75,5 MHz) : 149,9 (C7) ; 127,5 (C2) ; 124,3 (C2') ; 122,6 (C5') ; 122,4 (C3) ; 120,4 (C5) ; 114,6 (C4) ; 110,1 (C4') ; 107,3 (C5) ; 44,8 (C6) ; **MS(ESI+) :** 175,1 [M+H⁺] ; 197,0 [M+Na] ; 215,0 [M+K].

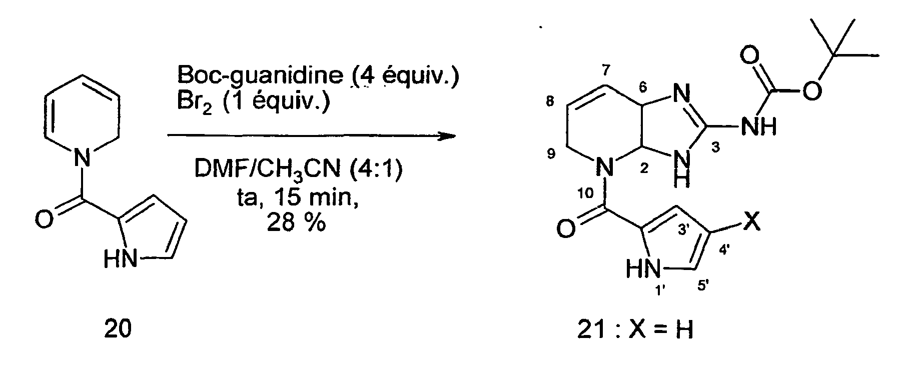

### Exemple 15 : acide [4-(1H-pyrrol-2-carbonyl)-3a,4,5,7a-tétrahydro-3H-imidazo[4,5-b]pyridin-2-yl]carbamique 21

La dihydropyridine **20** (0,50 g, 2,8 mmoles, 1 équiv.) et la N-Boc-guanidine (1,36 g, 8,4 mmoles, 3 équiv.) sont placées en suspension dans un mélange de DMF/CH₃CN : 3/1 (8 mL). A cette suspension est ajouté le brome (0,14 mL, 2,8 mmoles, 1 équiv.) en solution dans du DMF (2 mL) goutte à goutte sur une période de 15 min. Dès la fin de l'addition, le mélange réactionnel est concentré sous pression réduite, puis séché à la pompe. Le résidu est purifié par chromatographie sur colonne de gel de silice, élué par un mélange CH₂Cl₂ saturé en ammoniac/MeOH : 99/1. Le produit de couplage **21** est obtenu avec un rendement de 28 % (0,26 g).

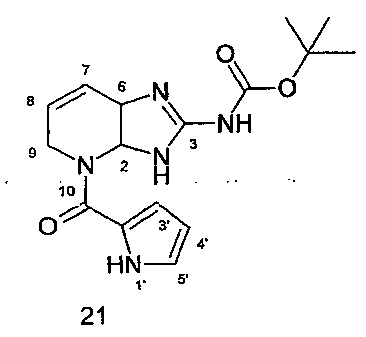

**RMN ¹H** (300 MHz, CD₃OD) : 6,97 (m, 1H, H5') ; 6,90 (d, *J* = 8,0 Hz, 1H, H2) ; 6,68 (m, 1H, H3') ; 6,24 (dd, *J* = 2,4 et 3,5 Hz, 1H, H4') ; 5,86 (dd, *J* = 4,0, 10 Hz, 1H, H8) ; 5,70 (dl, *J* = 10,0 Hz, 1H, H7) ; 4,66 (m, 1H, H9) ; 4,59 (m, 1H, H6) ; 4,25 (m, 1H, H9) ; **MS(ES) :** 354,15 [M+Na⁺] ; 197,0 [M+Na⁺] ; 332,15 [M+H⁺] ; 232,08 [M-Boc]⁺.

### Exemple 16 : ester tertio-butylique de l'acide [4-(4-dibromo-1H-pyrrol-2-carbonyl)-3a,4,5,7a-tétrahydro-3H-imidazo[4,5-b]pyridin-2-yl]carbamique 22

Même mode opératoire que pour **21**, mais avec 2 équiv. de Br₂.(rendement optimisé de 19%)

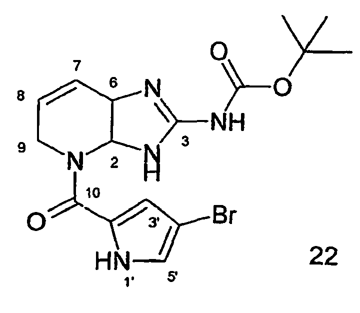

**RMN ¹H** (300 MHz, CD₃OD) : 7,00 (d, *J* = 1,0 Hz, 1H, H5') ; 6,84 (d, *J* = 8,5 Hz, 1H, H2) ; 6,72 (m, 1H, H3') ; 5,85 (dd, *J* = 4,8, 9,6 Hz, 1H, H7) ; 5,72 (dl, *J* = 10,3 Hz, 1H, H8) ; 4,60 (dd, *J* = 4,7, 17,5 Hz, 1H, H6) ; 4,27 et 4,03 (2m, 2H, H9) ; **RMN ¹³C** (75,5 MHz) : 28,1 ((CH₃)₃) ; 40,0 (C9) ; 52,6 (C6) ; 79,4 (CtBu) ; 8,6 (C2) ; 97,5 (C4') ; 115,6 (C8) ; 123,6 (C3') ; 124,8 (C7) ; 126,5 (C7) ; 127,7 (C5') ; 146,3 et 167,0 (C10 et CO carbamate) ; **SM(ES) :** 412,1-410,1 [M+H⁺], 332,2 [M-HBr]⁺, 312,0-310,0 [M-Boc]⁺, 232,1 [M-2HBr]⁺.

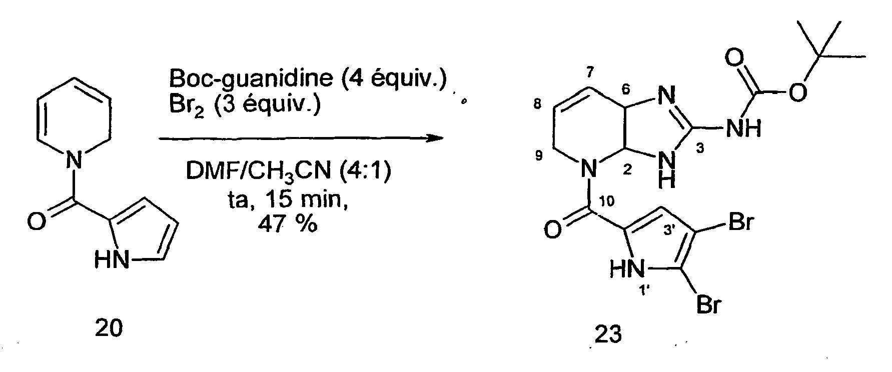

### Exemple 17 : ester tertio-butylique de l'acide [4-(4,5-dibromo-1H-pyrrol-2-carbonyl)-3a,4,5,7a-tétrahydro-3H-imidazo[4,5-b]pyridin-2-yl]carbamique 23

Le composé **20** (0,24 g, 1,4 mmoles, 1 équiv.) et la N-Boc-guanidine (0,65 g, 4,1 mmoles, 4 équiv.) sont placées en suspension dans un mélange DMF/CH₃CN : 3/1 (8 mL). Le brome (0,21 mL, 4,2 mmoles, 3 équiv.) en solution dans du DMF (2 mL) est ajoutée goutte à goutte sur une période de 15 min. Dès la fin de l'addition, le mélange réactionnel est concentré sous pression réduite et séché à la pompe. Le résidu est purifié par chromatographie sur colonne de gel de silice, élué par un mélange CH₂Cl₂ saturé en ammoniac/MeOH : 99/1. Le produit **23** est obtenu avec un rendement de 47 % (0,31 g).

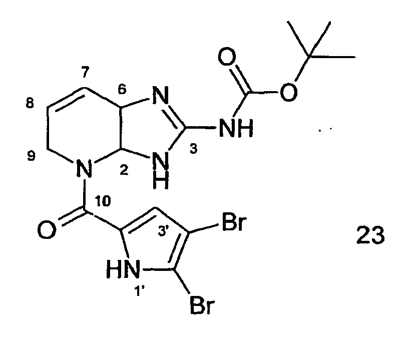

**RMN ¹H** (300 MHz, CD₃OD) : 6,84 (s, 1H, H3') ; 6,67 (d, *J* = 8,5 Hz, 1H, H2) ; 6,17 (td, *J* = 2,5 et 10,5 Hz, 1H, H8) ; 5,80 (dd, *J* = 1,6 et 10,5 Hz, 1H, H7) ; 4,62 (d, 1H, H6) ; 4,53 et 4,03 (2m, 2H, H9) ; **RMN ¹³C** (75,5 MHz) : 27,8 ((CH₃)₃) ; 41,7 (C9) ; 53,1 (C6) ; 76,1 (CtBu) ; 84,7 (C2) ; 95,8 (C4') ; 97,2 (C3') ; 103,8 (C5') ; 114,9 (C8) ; 124,4 (C2') ; 126,5 (C7) ; 152,2 et 161,8 (C10 et CO carbamate) ; **SM(ES) :** 490,0 [M+H⁺], 410,1 [M-HBr]⁺, 389,9 [M-Boc]⁺, 332,1 [M-2HBr]⁺, 310,0 [M-HBr-Boc]⁺, 232,1 [M-2HBr-Boc]⁺.

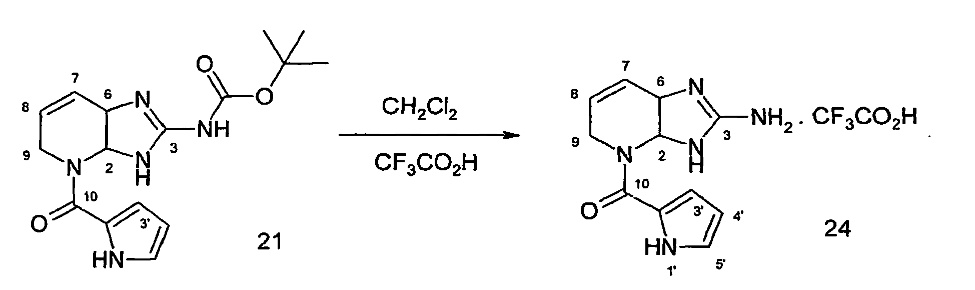

### Exemple 18 : acide [4-(1H-pyrrol-2-carbonyl)-3a,4,5,7a-tétrahydro-3H-imidazo[4,5-b]pyridin-2-yl]carbamique 24

Le composé **21** (0,15 g, 0,3 mmoles, 1 équiv.) est solubilisé dans du dichlorométhane (3 mL), puis additionné du même volume d'acide trifluoroacétique. Le mélange réactionnel est agité à température ambiante jusqu'à consommation totale du produit de départ. Le mélange est alors concentré sous pression réduite et séché à la pompe pendant 2 h. Le résidu est purifié par chromatographie sur colonne de gel de silice, élué par un mélange CH₂Cl₂ saturé en ammoniac/MeOH : 9/1. Le produit **24** est obtenu avec un rendement de 68 % (0,11 g).

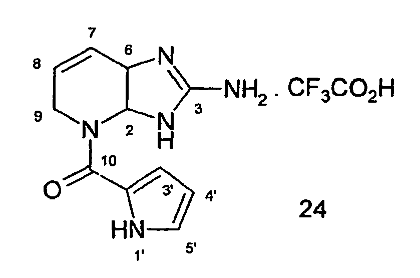

**RMN ¹H** (300 MHz, CD₃OD) : 6,99 (m, 1H, H5') ; 6,81 (d, *J* = 8,3 Hz, 1H, H2) ; 6,73 (dd, *J* = 1,2 et 3,6 Hz, 1H, H3') ; 6,24 (dd, *J* = 2,4, 3,6 Hz, 1H, H4') ; 6,5 (ddd, *J* = 1,9, 5,0 et 10,3 Hz, 1H, H8) ; 5,76 (dd, *J* = 1,8 et 10,3 Hz, 1H, H7) ; 4,67 (dd, *J* = 5,0 et 18,0 Hz, 1H, H9) ; 4,52 (d, *J* = 8,4 Hz, 1H, H6) ; 3,95 (d, *J* = 18,0 Hz, 1H, H9) ; **RMN ¹³C** (75,5 MHz) : 39,4 (C9) ; 50,1 (C6) ; 64,9 (C2) ; 108,7 (C4') ; 113,3 (C3') ; 121,5 (C8) ; 122,2 (C5') ; 122,7 (C2') ; 126,5 (C7) ; 158,7 (C10) ; 163,5 (C4).

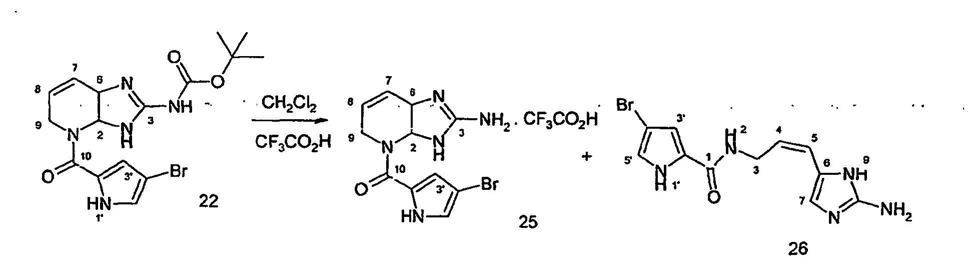

### Exemple 19 : Acide [4-(1H-pyrrol-2-carbonyl)-3a,4,5-7a-tétrahydro-3H-imidazo[4,5-b]pyridin-2-yl]carbamique 25 et son produit d'ouverture 26

Le bicycle 22 (50 mg, 0,12 mmole, 1 équiv.) est solubilisé dans du dichlorométhane (1 mL). Un volume équivalent d'acide trifluoroacétique lui est ajouté et le milieu est agité à température ambiante jusqu'à consommation totale du produit de départ. Le mélange réactionnel est alors concentré sous pression réduite et séché à la pompe pendant 2 h. Le résidu est purifié par chromatographie sur couche mince préparative, élué par un mélange CH₂Cl₂ saturé en ammoniac/MeOH : 9/1. Les produits **25** et **26 (*cis*-hyménidine) :** sont obtenus avec un rendement de 52 % et 15 % respectivement.

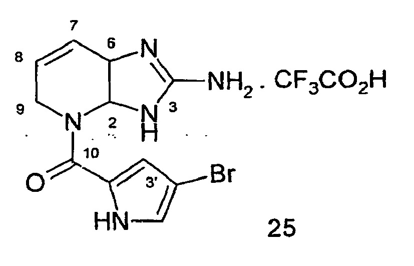

**RMN ¹H** (300 MHz, CD₃OD) : 7,02 (d, *J* = 1,2 Hz, 1H, H5') ; 6,76 (d, *J* = 1,2 Hz, 1H, H3') ; 6,73 (d, *J* = 1,2 Hz, 1H, H2) ; 6,15 (ddd, *J* = 1,7 ; 4,8 et 10,3 Hz, 1H, H8) ; 5,77 (dd, *J* = 2,0 et 10,3 Hz, 1H, H7) ; 4,65 (dd, *J* = 4,8, 17,8 Hz, 1H, H9) ; 4,53 (d, *J* = 8,2 Hz, 1H, H6) ; 3,96 (d, *J* = 18,0 Hz, 1H, H9) ; **RMN ¹³C** (75,5 MHz) : 40,9 (C9) ; 51,8 (C6) ; 66,5 (C2) ; 97,6 (C4') ; 116,2 (C3') ; 123,1 (C8) ; 123,8 (C5') ; 125,1 (C2') ; 128,0 (C7) ; 160,3 (C10) ; 163,1 (C4) ; **SM(ES) :** 312,0-310,0 [M+H⁺], 232,1 [M-HBr]⁺.

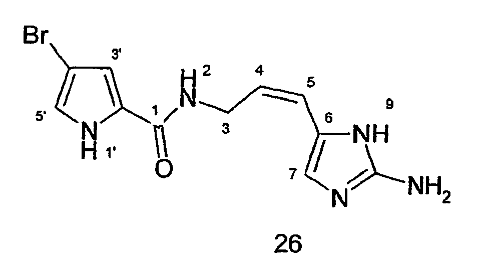

**RMN ¹H** (300 MHz, CD₃OD) : 6,90 (d, *J* = 1,4 Hz, 1H, H5') ; 6,77 (d, *J* = 1,4 Hz, 1H, H3') ; 6,59 (s, 1H, H6) ; 6,20 (d, *J* = 11,6 Hz, 1H, H5) ; 5,48 (dt, *J* = 6,7 et 11, 6 Hz, 1H, H4) ; 4,16 (dd, *J* = 1,3 et 6,7 Hz, 2H, H3) ; **RMN ¹³C** (75,5 MHz) : 39,5 (C3) ; 97,65 (C4') ; 113,5 (C3') ; 118,1 (C7) ; 121,4 (C5) ; 123,0 (C4) ; 125,5 (C5') ; 125,9 (C2').

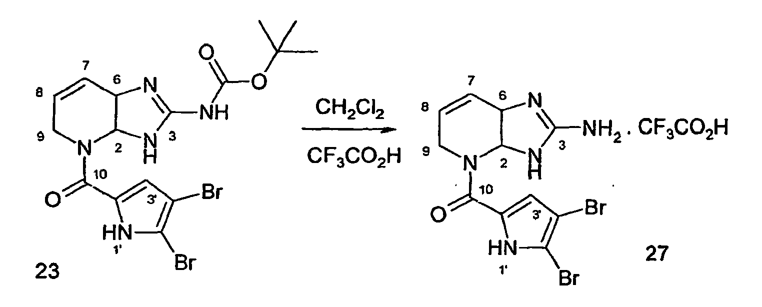

### Exemple 19 : Acide [4-(4,5-dibromo-1H-pyrrol-2-carbonyl)-3a,4,5,7a-tétrahydro-3H-imidazo[4,5-b]pyridin-2-yl]carbamique 27

Le composé **23** (30 mg, 0,06 mmole, 1 équiv.) est solubilisé dans du dichlorométhane (2 mL). Un volume équivalent d'acide trifluoroacétique est ajouté et le milieu est agité à température ambiante jusqu'à consommation totale du produit de départ. Le mélange est alors concentré sous pression réduite et séché à la pompe pendant 2 h. Le résidu est purifié par plaques préparatives éluées par un mélange CH₂Cl₂ saturé en ammoniac/MeOH : 9/1. Le produit déprotégé **27** est obtenu avec un rendement de 53 % (16 mg). 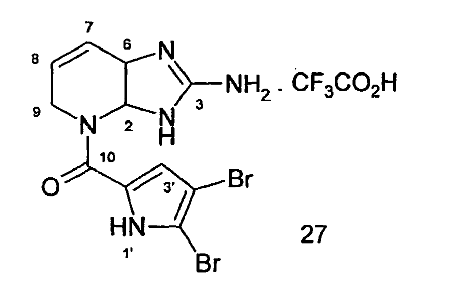 **RMN ¹H** (300 MHz, CD₃OD) : 6,80 (s, 1H, H3') ; 6,70 (d, *J* = 8,6 Hz, 1H, H2) ; 6,15 (ddd, *J* = 2,2 ; 5,0 et 10,3 Hz, 1H, H8) ; 5,76 (dd, *J* = 1,8 et 10,3 Hz, 1H, H7) ; 4,62 (dd, *J* = 1,8, 10,3 Hz, 1H, H9) ; 4,54 (dd, *J* = 2,7 Hz, 1H, H6) ; 3,98 (d, *J* = 16,0 Hz, 1H, H9) ; **SM(ESI+) :** 392,0-390,0-388,0 [M+H⁺] ; 312,0-310,0 [M-HBr]⁺; 232,1 [M-2HBr]⁺.

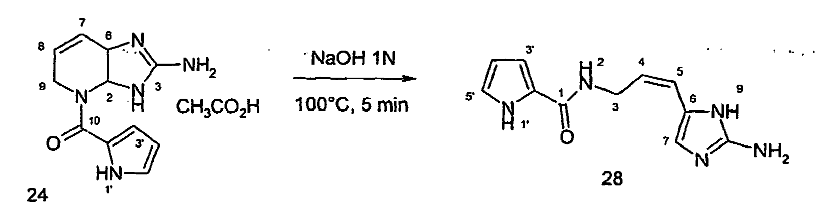

### Exemple 20 : la cis-clathrodine 28

Le bicycle **24** (100 mg, 0,3 mmole, 1 équiv.) est placé en suspension dans une solution de soude 1N (2 mL) et chauffé à 100°C pendant 5 min. Après retour à température ambiante, le milieu réactionnel est extrait par l'acétate d'éthyle (4x5 mL). La phase organique est séchée sur sulfate de magnésium et concentrée sous pression réduite. La clathrodine **28** est obtenue avec un rendement non optimisé de 25 % (17 mg).

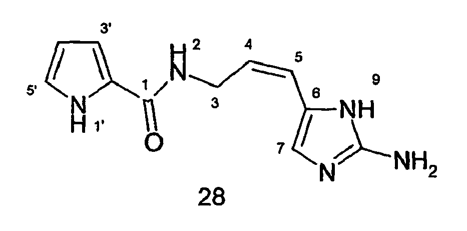

**RMN ¹H** (300 MHz, CD₃OD) : 6,90 (m, 1H, 5'H) ; 6,76 (dd,1H *J* = 1,4, 3,7 Hz, 1H, H3') ; 6,65 (s, 1H, H6) ; 6,21 (dd, *J* = 11, 7 Hz, 1H, H5) ; 6,15 (m, 1H, H4') ; 5,56 (dt, *J* = 6,8, 11,7 Hz, 1H, H4) ; 4,14 (dd, *J* = 1,7 et 6,8 Hz, 2H, H3) ; **RMN ¹³C** (75,5 MHz) : 163,8 (C8) ; 151,3 (C1) ; 129,9 (C6) ; 126,8 (C2') ; 125,0 (C5') ; 122,9 (C4) ; 121,3 (C5) ; 118,6 (C7) ; 111,8 (C3') ; 110,2 (C4') ; 39,3 (C3).

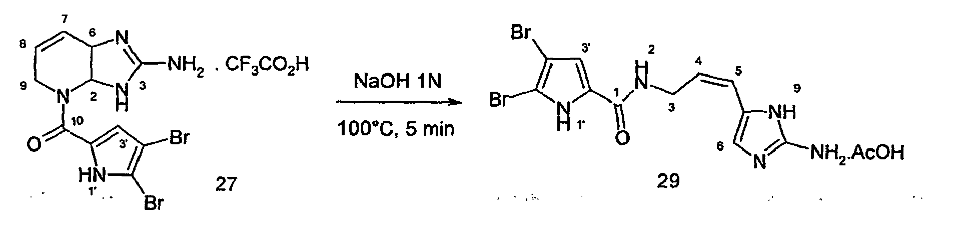

### Exemple 21 : synthèse de la cis-oroidine 29

Le bicycle **27** (16 mg, 0,03 mmole, 1 équiv.) est placé en suspension dans une solution de soude 1N (1 mL) et chauffé à 100°C pendant 10 min. Après retour à température ambiante, le milieu est extrait par l'acétate d'éthyle (5x5 mL). La phase organique est séchée sur sulfate de magnésium et concentrée sous pression réduite, resolubilisée dans quelques gouttes de méthanol et une goutte d'acide acétique, puis séchée. Le sel du produit **29** est obtenu avec un rendement de 85 % (13 mg).

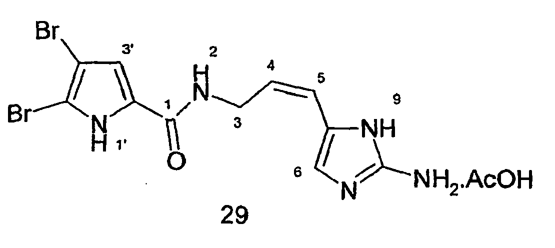

**RMN ¹H** (300 MHz, CD₃OD) : 6,82 (s, 1H, H3') ; 6,79 (s, 1H, H6) ; 6,20 (d, *J* = 11,5 Hz, 1H, H5) ; 5,74 (td, *J* = 7,1 et 11,5 Hz, 1H, H4) ; 4,08 (d, *J* = 7,1 Hz, 2H, H3) ; 1,96 (s, 3H, AcOH) ; **SM(ES) :** 391,96-389,97-387,97 (M+H)⁺ ; 310,04 (M-HBr)⁺ ; 232,10 (M-2HBr)⁺.

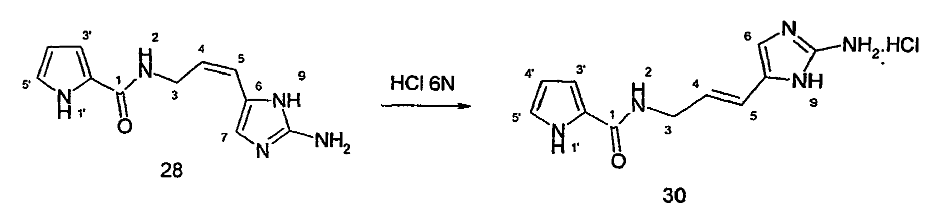

### Exemple 22 : la clathrodine 30

La *cis*-clathrodine.AcOH **28** (24 mg, 0,08 mmole, 1 équiv.) est solubilisée dans du méthanol (0, 5 mL), puis additionnée d'une solution d'HCl 6N (0,6 mL). Le milieu réactionnel est chauffé à 60°C pendant 24 h. Après retour à température ambiante et concentration à sec du mélange sous pression réduite, le résidu est traité par plaques préparatives, éluées par un mélange CH₂Cl₂ saturé en ammoniac/MeOH : 9/1. Le produit **30** est obtenu avec un rendement de 24 % (5 mg). Les caractéristiques physiques sont identiques à celles de la littérature.

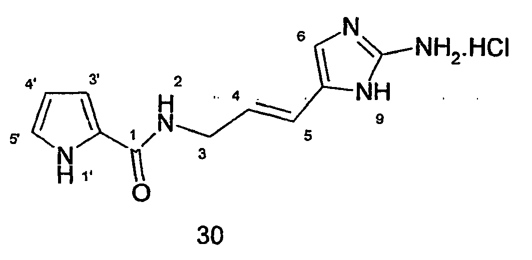

**RMN ¹H** (300 MHz) : 6,90 (m, 1H, H5') ; 6,79 (dd, *J* = 1,4 et 3,5 Hz, 1H, H3') ; 6,46 (s, 1H, H6) ; 6,32-6,26 (d, *J* = 15,8 Hz, 1H, H5) ; 6,15 (dd, *J* = 2,4 et 3,4 Hz, 1H, H4') ; 5,90 (dt, *J* = 6,1 et 15,8 Hz, 1H, H4) ; 4,02 (d, *J* = 6,1 Hz, 2H, H3).

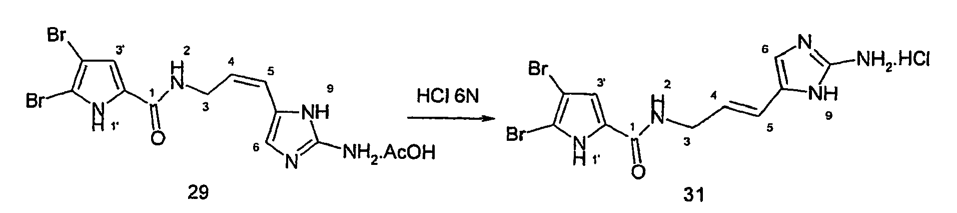

### Exemple 23 : synthèse de l'oroidine 31

La *cis*-oroidine.AcOH **29** (12 mg, 0,03 mmole, 1 équiv.) est solubilisée dans du méthanol (0,4 mL), puis additionnée de 0,6 mL d'une solution d'HCl 6N, et le milieu est chauffé à 60°C pendant 24 h. Après retour à température ambiante, le milieu est concentré sous pression réduite et séché à la pompe. Le résidu est purifié par plaques préparatives, éluées par un mélange CH₂Cl₂ saturé en ammoniac/MeOH : 9/1. L'oroidine **31** est obtenue avec un rendement de 44 % (5 mg). Les caractéristiques physiques sont identiques à celles de la littérature

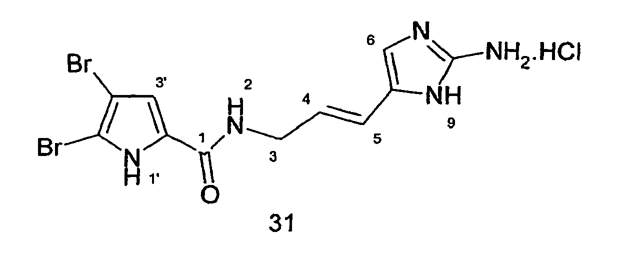

**RMN ¹H** (300 MHz, CD₃OD) : 6,82 (s, 1H, H3') ; 6,49 (s, 1H, H6) ; 6,31-6,26 (d, *J* = 15,7 Hz, 1H, H5) ; 5,92 (td, *J* = 6,0, 15,7 Hz, 1H, H4) ; 4,0 (d, *J* = 6,0 Hz, 2H, H3).

### Exemple 24 : 2-imino-2,3,5,6-tétrahydro-1H-1,3,5-triaza-azulèn-4-one

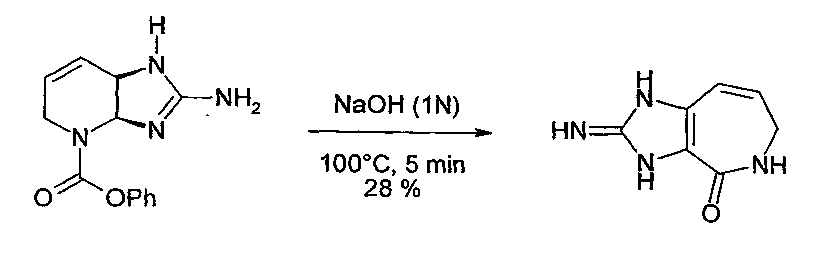

A 4 ml d'une solution NaoH 1M, on ajoute le composé 10 (0,134g, 0,4 mmole) du chlorhydrate du méthylate de l'acide 2-Amino-1,3a,5,7a-tetrahydro-imidazo[4,5-b]pyridine-4-carboxylique. Le mélange réactionnel est porté à reflux sous agitation pendant 5 minutes (réaction contrôlée par CCM). Après refroidissement, on ajoute de l'eau (20 mL), et le mélange est extrait au BuOH jusqu'à épuisement. Les phases organiques sont regroupées et évaporées à sec pour donner 0,467 g d'un brut qui après séparation par plaques de silice à l'aide du mélange ACUE/Acétone/HCO₂H/H₂O : 5/3/0,5/0,5) donne 40 mg de **32** (0,24 mmole), soit un rendement de 28 %.

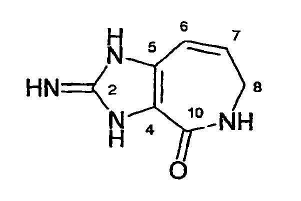

### 2-imino-2,3,5,6-tétrahydro-1H-1,3,5-triaza-azulèn-4-one

**RMN ¹H** (300 MHz, CD₃OD) : δ = 3,48 (d, *J* = 6 Hz, 2H, H-8), 5,88 (dt, *J* = 6 et 11 Hz, 1H, 7-H), 6,52 (d, *J* = 11 Hz, 1H, 6-H). **RMN ¹³C** (75,5 MHz, CD₃OD) : δ 38,4 (C-8), 124,6 (C-7), 126,5 (C-6), 159,7 (C-2), 162,9 (C-10). **SM(ES) :** *m*/*z* = 165 [M+H]⁺, 187,0526 [M+Na]⁺; **HRSM(ES) :** calculé pour C₇H₈N₄O : 165,0776 ; trouvé 165.0785 IR(cm⁻¹) CHCl₃ : 2928, 1669, 1630, 1545, 1079, 786.

## Revendications

1. Procédé de synthèse de composés hétérocycliques, **caractérisé en ce qu'**il comprend l'ouverture d'un composé de formule I : 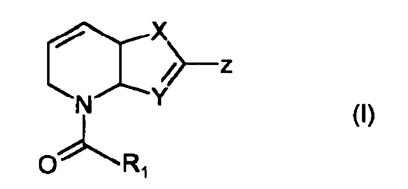 dans laquelle,
- X représente NH, O, S ou un groupe N-p, p étant un groupe protecteur tel que Boc ou Troc,
- Y représente N, O, S,
- Z représente NH₂ ou NH-p, et
- R₁ représente un radical alkoxy en C₁-C₆, aryloxy, notamment phényloxy, ou un radical pyrrolyle, ces radicaux étant éventuellement substitués, ou de leurs sels, et des isomères de ces composés,
ladite étape d'ouverture étant réalisée dans des conditions conduisant à un hétérocycle de formule II : 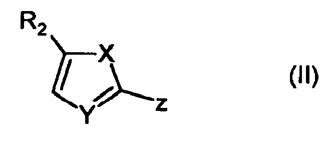 dans laquelle,
- X, Y et Z sont tels que définis ci-dessus, et
- R₂ représente un groupe -CH=CH-CH₂-NH-COR₁ ou 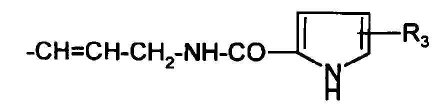
- R₃ occupant une, deux ou trois positions et représentant un halogène.

2. Procédé selon la revendication 1, **caractérisé en ce que** l'étape d'ouverture du bicycle est réalisée en milieu alcalin, ou dans un solvant tel que le DMSO, en chauffant au reflux.

3. Procédé selon la revendication 1, **caractérisé en ce que,** lorsque R₁ représente un groupe pyrrolyle, le cas échéant substitué, on fait réagir le bicycle en solution dans un solvant tel que le dichlorométhane avec de l'acide trifluroacétique, à température ambiante.

4. Procédé selon l'une quelconque des revendications 1 à 3, **caractérisé en ce que** l'étape d'ouverture est réalisée sur un mélange de régioisomères de formule I.

5. Procédé selon l'une quelconque des revendications 1 à 3, **caractérisé en ce que** l'on utilise un sel d'un composé de formule I.

6. Procédé selon l'une quelconque des revendications 1 à 5, **caractérisé en ce que** les composés de formule I sont des hydro-imidazo-pyridines.

7. Procédé selon la revendication 6, **caractérisé en ce qu'**il s'agit d'hydro-imidazo-pyridines de formule III : 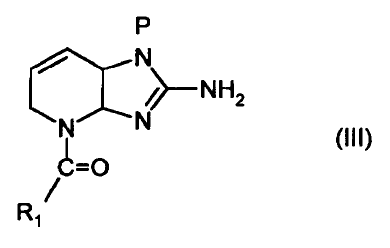 ou de formule IV :

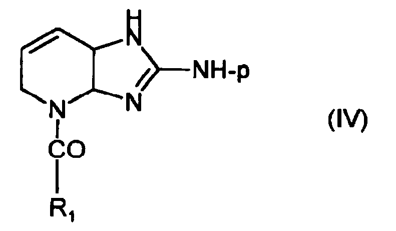

8. Procédé selon la revendication 7, **caractérisé en ce que** l'étape d'ouverture est réalisée sur un sel d'hydro-imidazo-pyridines de formule V ou VI :

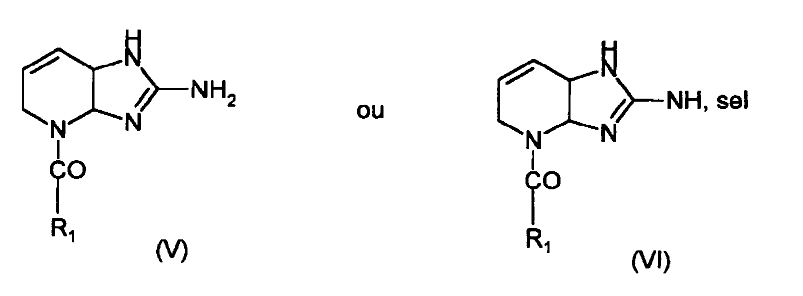

9. Procédé selon l'une quelconque des revendications 1 à 5, **caractérisé en ce que** les composés de formule I soumis à l'étape d'ouverture sont des hydro-oxazo-pyridines.

10. Procédé selon la revendication 9, **caractérisé en ce que** les hydro-oxazo-pyridines répondent à la formule VII :

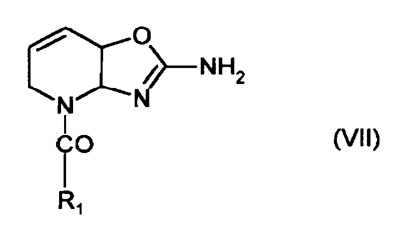

11. Procédé selon la revendication 10, **caractérisé en ce qu'**en portant à reflux une solution desdits composés dans un solvant tel que le DMF, on obtient des bicycles de formule VIII :

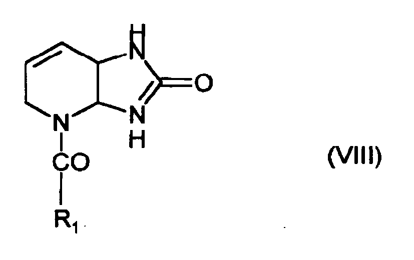

12. Procédé selon l'une quelconque des revendications 1 à 5, **caractérisé en ce que** les composés de formule I sont des hydro-thiazo-pyridines.

13. Procédé selon l'une quelconque des revendications 1 à 12, **caractérisé en ce que** les bicycles de formule I sont obtenus par réaction d'une dihydropyridine de formule IX : 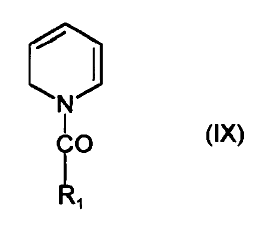 avec un dérivé de formule X : 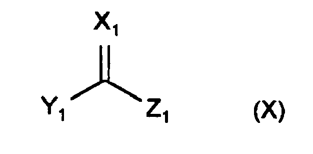 dans laquelle :
- X₁ représente O, S ou NH, et
- Y₁ et Z₁ représentent NH₂.

14. Procédé selon la revendication 13, **caractérisé en ce que** la réaction entre les composés de formules IX et X est réalisée en présence de brome dans des solvants organiques tels que DMF et/ou CH₃CN, à température ambiante et sous argon.

15. En tant que nouveaux produits, les composés de formule II.

16. En tant qu'intermédiaires, les composés de formule I tels que définis ci-dessus.

## Claims

1. Method for synthesising heterocyclic compounds, **characterised in that** it comprises the opening of a formula I compound: 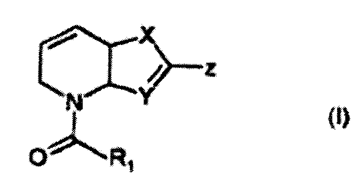 in which,
- X represents NH, O, S or a N-p group, p being a protector group such as Boc or Troc,
- Y represents N, O, S,
- Z represents NH₂ or NH-p, and
- R₁ represents a C₁-C₆ alkoxy radical, aryloxy, in particular phenyloxy, or a pyrrolyle radical, these radicals being optionally substituted, or their salts, and isomers of these compounds,
said opening step being carried out under conditions leading to a formula II heterocycle: 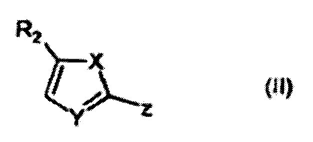 in which,
- X, Y and Z are as defined above, and
- R₂ represents a -CH=CH-CH₂-NH-COR₁ group or 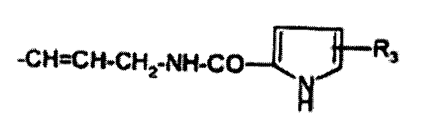
- R₃ occupying one, two or three positions and representing a halogen.

2. Method according to claim 1, **characterised in that** the bi-cycle opening step is carried out in alkaline medium, or in a solvent such as DMSO, by heating under reflux.

3. Method according to claim 1, **characterised in that,** when R₁ represents a pyrrolyle group, optionally substituted, the bicycle is reacted in solution in a solvent such as dichloromethane with trifluoroacetic acid, at room temperature.

4. Method according to any of claims 1 to 3, **characterised in that** the opening step is carried out on a mixture of formula I regioisomers.

5. Method according to any of claims 1 to 3, **characterised in that** a salt of a formula I compound is used.

6. Method according to any of claims 1 to 5, **characterised in that** the formula I compounds are hydro-imidazo-pyridines.

7. Method according to claim 6, in these are formula III hydro-imidazo-pyridines: 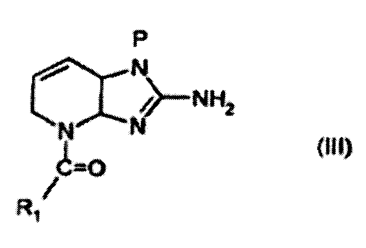 or formula IV hydro-imidazo-pyridines:

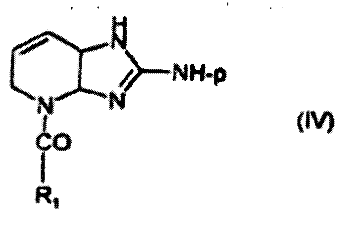

8. Method according to claim 7, **characterised in that** the opening step is carried out on a formula V or formula VI hydro-imidazo-pyridine salt:

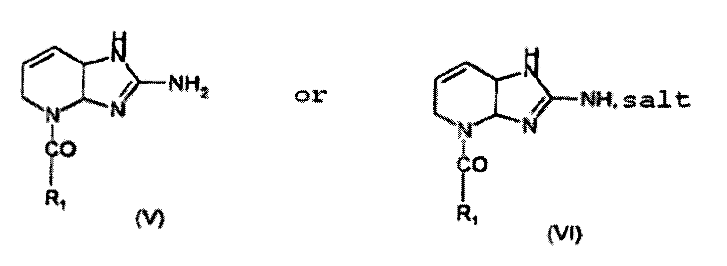

9. Method according to any of claims 1 to 5, **characterised in that** the formula I compounds subjected to the opening step are hydro-oxazo-pyridines.

10. Method according to claim 9, **characterised in that** the hydro-oxazo-pyridines have formula VII:

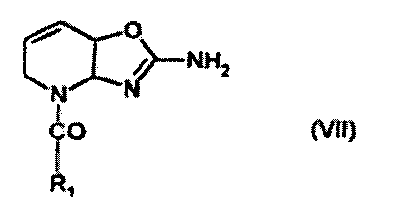

11. Method according to claim 10, **characterised in that** while heating under reflux a solution of said compounds in a solvent such as DMF, formula VIII bicycles are obtained:

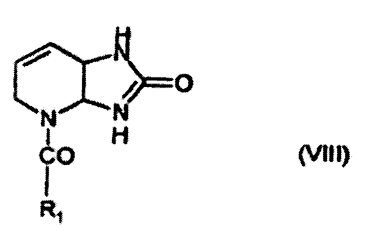

12. Method according to any of claims 1 to 5, **characterised in that** the formula I compounds are hydro-thiazo-pyridines.

13. Method according to any one of claims 1 to 12, **characterised in that** the formula I bicycles are obtained by reacting a formula IX dihydropyridine: 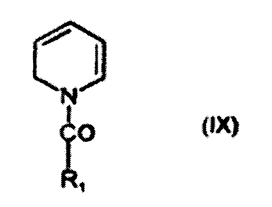 with a formula X derivative: 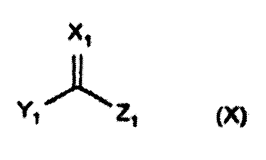 in which:
- X₁ represents O, S or NH, and
- Y₁ and Z₁ represent NH₂.

14. Method according to claim 13, **characterised in that** the reaction between the formula IX and formula X compounds takes place in the presence of bromine in organic solvents such as DMF and/or CH₃CN, at room temperature and under argon.

15. As new products, formula II compounds.

16. As intermediaries, formula I compounds as defined above.

## Patentansprüche

1. Syntheseverfahren heterozyklischer Verbindungen, **dadurch gekennzeichnet, dass** es die Öffnung einer Verbindung der Formel I umfasst: 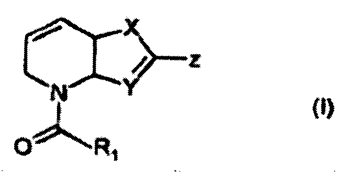 worin:
- x NH, O, S oder einer Gruppe N-p entspricht, wobei p einer Schutzgruppe wie z. B. Boc oder Troc entspricht,
- Y N, O, S entspricht,
- Z NH₂ oder NH-p entspricht, und
- R₁ einem Alkoxy-Radikal in C₁-C₆, Aryloxy, besonders Phenyloxy, oder einem Pyrrolyl-Radikal, wobei diese Radikale ersetzt werden können, oder deren Salzen und den Isomeren dieser Verbindungen entspricht,
wobei diese Öffnungsphase unter solchen Bedingungen erfolgt, die zu einem Heterozyklus der Formel II führen: 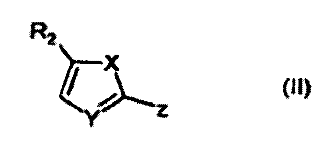 worin:
- X, Y und Z obiger Definition entsprechen, und
- R₂ einer Gruppe -CH=CH-CH₂-NH-COR₁ bzw. 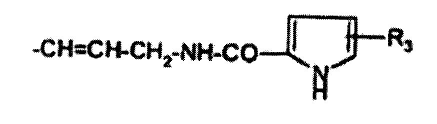 entspricht
- R₃ eine, zwei oder drei Positionen belegt und einem Halogen entspricht.

2. Verfahren nach Anspruch 1, **dadurch gekennzeichnet, dass** die Öffnungsphase des Bizyklus in einer alkalischen Umgebung bzw. in einer Lösung wie z. B. DMSO und mit Erhitzung des Rückstroms erfolgt.

3. Verfahren nach Anspruch 1, **dadurch gekennzeichnet, dass** wenn R₁ einer gegebenenfalls ersetzten Pyrrolyl-Gruppe entspricht, die Reaktion des Bizyklus gelöst in einem Lösungsmittel wie z. B. Dichlormethan mit der Säure Trifluorsäure bei Umgebungstemperatur ausgelöst wird.

4. Verfahren nach einem der Ansprüche 1 bis 3, **dadurch gekennzeichnet, dass** die Öffnungsphase auf einem Regioisomer-Gemisch nach der Formel I erfolgt.

5. Verfahren nach einem der Ansprüche 1 bis 3, **dadurch gekennzeichnet, dass** man das Salz einer Verbindung nach der Formel I verwendet.

6. Verfahren nach einem der Ansprüche 1 bis 5, **dadurch gekennzeichnet, dass** es sich bei den Verbindungen der Formel I um Hydro-Imidazopyridine handelt.

7. Verfahren nach Anspruch 6, **dadurch gekennzeichnet, dass** es sich um Hydro-Imidazopyridine nach der Formel III: 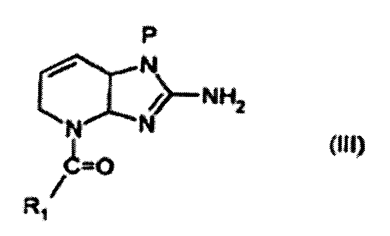 oder nach der Formel IV handelt:

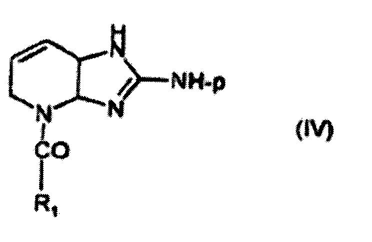

8. Verfahren nach Anspruch 7, **dadurch gekennzeichnet, dass** die Öffnungsphase auf einem Salz der Hydro-Imidazopyridine nach der Formel V bzw. VI erfolgt:

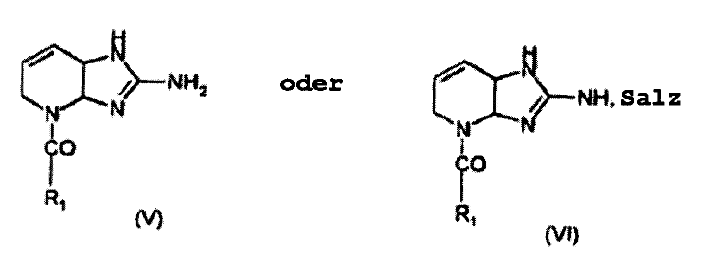

9. Verfahren nach einem der Ansprüche 1 bis 5, **dadurch gekennzeichnet, dass** es sich bei den Verbindungen der Formel I, die dem Öffnungsprozess unterzogen werden, um Hydro-Oxazopyridine handelt.

10. Verfahren nach Anspruch 9, **dadurch gekennzeichnet, dass** die Hydro-Oxazopyridine der Formel VII entsprechen:

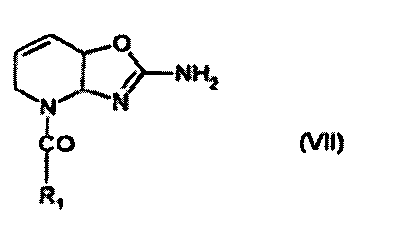

11. Verfahren nach Anspruch 10, **dadurch gekennzeichnet, dass** durch den Rückstrom einer Lösung dieser Verbindungen in einem Lösungsmittel wie z. B. DMF Bizyklen nach der Formel VIII erhalten werden:

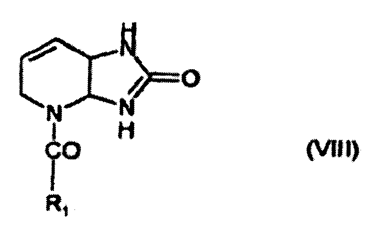

12. Verfahren nach einem der Ansprüche 1 bis 5, **dadurch gekennzeichnet, dass** es sich bei den Verbindungen der Formel I um Hydro-Thiazopyridine handelt.

13. Verfahren nach einem der Ansprüche 1 bis 12, **dadurch gekennzeichnet, dass** die Bizyklen der Formel I durch die Reaktion eines Dihydropyrindins nach der Formel IX: 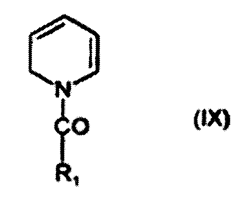 mit einem Derivat der Formel X erhalten werden: 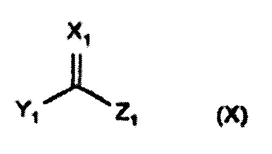 worin:
- X₁ O, S oder NH entspricht, und
- Y₁ und Z₁ NH₂ entsprechen.

14. Verfahren nach Anspruch 13, **dadurch gekennzeichnet, dass** die Reaktion zwischen den Verbindungen der Formeln IX und X in Anwesenheit von Brom in den organischen Lösungen wie z. B. DMF bzw. CH₃CN bei Umgebungstemperatur und unter Argon erfolgt.

15. Als neue Produkte, die Verbindungen nach der Formel II.

16. Als Zwischenprodukte, die Verbindungen der Formel I, wie sie weiter oben definiert werden.
